# EUROPEAN PATENT SPECIFICATION

(11) **EP 2 480 883 B1**
(45) Date of publication and mention of the grant of the patent: **01.04.2020**
(21) Application number: 10850395.4
(22) Date of filing: 23.09.2010
(51) Int. Cl.: C12Q 1/6883, G01N 33/68

(54) **METHODS FOR ASSESSING INFERTILITY AND/OR EGG QUALITY**
VERFAHREN ZUR ÜBERPRÜFUNG VON UNFRUCHTBARKEIT UND/ODER EIERQUALITÄT
PROCÉDÉS POUR ÉVALUER LA STÉRILITÉ ET/OU LA QUALITÉ DES OVULES

(30) Priority: 23.09.2009 US 245265 P; 19.04.2010 US 325810 P
(43) Date of publication of application: 01.08.2012
(73) Proprietor: Celmatix, Inc., New York, NY 10013 (US)
(72) Inventor: YURTTAS, Piraye, New York,NY 10003 (US); BANDAK, Laura, New York,NY 10013 (US)
(74) Representative: HGF Limited
(86) International application number: PCT/US2010/050063
(87) International publication number: WO 2011/133175

(56) References cited:
- EP-A1- 1 947 195
- WO-A1-2006/055761
- WO-A1-2009/109043
- US-A1- 2006 172 322
- MCKENZIE L J ET AL: "Human cumulus granulosa cell gene expression: a predictor of fertilization and embryo selection in women undergoing IVF", HUMAN REPRODUCTION, OXFORD JOURNALS, GB, vol. 19, no. 12, 1 December 2004 (2004-12-01), pages 2869-2874, XP008105175, ISSN: 0268-1161, DOI: 10.1093/HUMREP/DEH535 [retrieved on 2004-10-07]
- VAN MONTFOORT A P A ET AL: "Differential gene expression in cumulus cells as a prognostic indicator of embryo viability: a microarray analysis", MOLECULAR HUMAN REPRODUCTION, OXFORD UNIVERSITY PRESS, GB - BE, vol. 14, no. 3, 1 March 2008 (2008-03-01), pages 157-168, XP002615004, ISSN: 1360-9947, DOI: 10.1093/MOLEHR/GAM088 [retrieved on 2008-01-18]
- YURTTAS ET AL.: 'Role for PADI6 and the cytoplasmic lattices in ribosomal storage in oocytes and translational control in the early mouse embryo' DEVELOPMENT vol. 135, 26 May 2008, pages 2627 - 2636, XP008154132
- TIAN ET AL.: 'Gene Birth, Death, and Divergence: The Different Scenarios of Reproduction-Related Gene Evolution.' BIOLOGY OF REPRODUCTION. vol. 80, January 2009, pages 616 - 621, XP008154188
- PARK ET AL.: 'Genetic Approach to Identify Critical Factors for Mouse Early Embryogenesis.' INTEGRATIVE BIOSCIENCES. vol. 10, 2006, pages 41 - 47, XP008154785
- WANG H.-X. ET AL: "Connexin expression and gap junctional coupling in human cumulus cells: contribution to embryo quality", JOURNAL OF CELLULAR AND MOLECULAR MEDICINE, UNIVERSITY PRESS CAROL DAVILA, BUCHAREST, RO, vol. 13, no. 5, 1 May 2009 (2009-05-01), pages 972-984, XP009147646, ISSN: 1582-1838 [retrieved on 2008-05-24]
- LEO BREIMAN: "Bagging Predictors", MACHINE LEARNING, KLUWER ACADEMIC PUBLISHERS-PLENUM PUBLISHERS, NE, vol. 24, no. 2, 1 August 1996 (1996-08-01) , pages 123-140, XP019213305, ISSN: 1573-0565
- LINDSEY J.K. ET AL: "Choosing among generalized linear models applied to medical data", STATISTICS IN MEDICINE, vol. 17, 15 January 1998 (1998-01-15), pages 59-68, XP055304816, US ISSN: 0277-6715, DOI: 10.1002/(SICI)1097-0258(19980115)17:1<59:: AID-SIM733>3.0.CO;2-7
- HEFLER L A ET AL: "A model for predicting age at menopause in white women", FERTILITY AND STERILITY, ELSEVIER SCIENCE INC, NEW YORK, NY, USA, vol. 85, no. 2, 1 February 2006 (2006-02-01), pages 451-454, XP028061876, ISSN: 0015-0282, DOI: 10.1016/J.FERTNSTERT.2005.07.1300 [retrieved on 2006-02-01]
- B.C.J.M. Fauser ET AL: "Predictors of ovarian response: progress towards individualized treatment in ovulation induction and ovarian stimulation", HUMAN REPRODUCTION UPDATE, vol. 14, no. 1, 10 December 2007 (2007-12-10), pages 1-14, XP055352053, GB ISSN: 1355-4786, DOI: 10.1093/humupd/dmm034

## Description

### Field of the Invention

The invention generally relates to methods for assessing infertility.

### Background

Approximately one in seven couples have difficulty conceiving. Infertility may be due to a single cause in either partner, or a combination of factors (e.g., genetic factors, diseases, or environmental factors) that may prevent a pregnancy from occurring or continuing. Every woman will become infertile in her lifetime. On average, egg quality and number begins to decline precipitously at 35. However, a number of women are fertile well into their 40's, and some women experience this decline much earlier in life. Though, generally, advanced maternal age (35 and above) is associated with poorer fertility outcomes, there is no way of diagnosing egg quality issues in younger women or knowing when a particular woman will start to experience decline in her egg quality or reserve.

*In vitro* fertilization (IVF), a process in which egg cells are fertilized by sperm outside a woman's womb and then transferred into the womb, is a common procedure to assist women who have difficulty conceiving. However, approximately 15% of all women undergoing IVF therapy are never successful with their own eggs, even after repeated attempts. Thus, egg quality is a primary factor in determining whether and how a woman's own eggs should be used in the IVF process.

HEFLER L A ET AL: "A model for predicting age at menopause in white women", FERTILITY AND STERILITY, vol. 85(2), 2006 discloses a model to predict the age at natural menopause. Ten SNPs from seven estrogen-metabolising genes are analysed by sequencing-on-chip technology. The model also includes the number of full-term pregnancies, the BMI and a history of breast surgery. The SNPs are considered binary variables (see page 452, top of left column).

B.C.J.M. Fauser ET AL: "Predictors of ovarian response: progress towards individualized treatment in ovulation induction and ovarian stimulation", HUMAN REPRODUCTION UPDATE, vol. 14(1) 2007, pages 1-14 discloses that a prediction model for a patient's ovarian response to medication is established. The prediction model is used in predicting a particular outcome based on independent variables using regression analysis. The use of genetic factors, such as polymorphisms in FSHR, is also disclosed.

From the time a woman seeks medical assistance for difficulty conceiving, she and her partner are advised to undergo a number of diagnostic procedures to ascertain potential causes for why the couple is having difficulty conceiving. For the female, such procedures are highly invasive, costly, and time consuming. Thus, there is a need for faster, non-invasive methods of assessing infertility and assessing a woman's egg quality.

### Summary

### Accordingly, the invention provides a method

for assessing infertility and/or egg quality in a post-pubescent mammal sample excluding a human embryonic sample, the method comprising:
a) conducting a sequencing assay that detects variation in at least six infertility-associated maternal effect genes from Table 1:

| **Table 1 - Human Fertility-Related Genes (OMIM ref # / Alternative Designations** | | |
|---|---|---|
| PADI6 | Pla2g4c | PROKI |
| (610363) | (603602) | (606233) |
| NPM2 | TACC3 | PROKRI |
| (608073) | (605303) | (607122) |
| NLRP5 | C60RF221 | PROKR2 |
| (609658 / MATER) | (611687 / FILIA) | (607123) |
| SMARCA4 | TLE6 | DPPA5 |
| (607123/ Brgl) | (612399) | (611111) |
| OOEP | AURKB | ACTL6A |
| (611689/ FlopedIMOEP19) | (604970) | (604958) |
| HSFI | AURKA | CARMI |
| (140580) | (603072) | (603934) |
| ZARI | DDX20 | SPIN |
| (607520) | (606168) | (609936) |
| DPPA3 | FIGLA | OAS1 |
| (608408/ Stella) | (608697) | (164350) |
| DNMT1 | ZP1 | VIL2 |
| (126375) | (195000) | (123900) |
| CTCF | ZP2 | NLRP14 |
| (604167) | (182888) | (609665) |
| PMS2 | ZP3 | CD55 |
| (600259) | (182889) | (125240) |
| BNC1 | IL-10 | IFITM1 |
| (601930) | (124092) | (604456 / Fragi1is) |
| BUB1 | KDM1B | GDF3 |
| (602452) | (613081) | (606522) |
| GDF1 | REX 1 | CDX2 |
| (602880) | (609614) | (600297) |
| BMP4 | KLF4 | EZH2 |
| (112262) | (602253) | (601573) |
| SUZ12 | SOX17 | MYC |
| (606245) | (610928) | (190080) |
| P53 | DDX43 | C60RF221 |
| (191170) | (606286) | (611687) |
| BRCA1 | EEF1A1 | BRCA2 |
| (113705) | (130590) | (600185) |
| FMR1 (309550) | DIAPH2 (300108) | HS6ST1 (604846) |
| GPC3 | FOXL2 | GALT |
| (300037) | (605597) | (606999) |
| FMR2 | BMP15 | FSHR |
| (300806) | (300247) | (136435) |
| LHCGR | FSHB | UBE3A |
| (152790) | (136530) | (601623) |
| LHB | INHA | AIRE |
| (152780) | (147380) | (607358) |
| EIF2B2 | EIF2B4 | EIF2B5 |
| (606454) | (606687) | (603945) |
| NOG | BMP7 | POLG |
| (602991) | (112267) | (174763) |
| USP9X | XPNPEP2 | ZFX |
| (300072) | (300145) | (314980) |
| CENPI | MADILI | CENPF |
| (300065) | (602686) | (600236) |
| MAD1L2 | XIST | CDX4 |
| (601467) | (314670) | (300025) |
| WT1 | ATM | ATR |
| (607102) | (607585) | (601215) |
| GDF9 | NOBOX | RFPL4A |
| (601918) | (610934) | (612601) |
| GREM2 | FOXE1 | BAX |
| (608832) | (602617) | (600040) |
| AHR | | |
| (600253) | | |

b) determining at least one infertility-associated phenotypic trait of the post-pubescent mammal or environmental exposure at least one of which is selected from Table 2:

| **Table 2 - Phenotypic and environmental variables impacting fertility success** |
|---|
| Cholesterol levels on different days of the menstrual cycle |
| Age of first menses for patient, sisters, mother, grandmothers |
| Age of menopause for sisters, mother, grandmothers |
| Number of previous pregnancies (biochemical/ectopic/clinical/fetal heart beat detected), age at the time, and outcome for patient, sisters, mother, grandmothers |
| Polycystic ovarian syndrome |
| History of hydrosalpinx or tubal occlusion |
| History of endometriosis or painful periods |
| Cancer history/type of cancer/treatment/outcome for patient, sisters, mother, grandmothers |
| Age that sexual activity began, current level of sexual activity |
| Smoking history for patient, sisters, mother, grandmothers |
| Travel schedule/number of flying hours a year/time difference changes of more than 3 hours (jetlag) |
| Nature of period (length of menses, length of cycle) |
| Biological age (number of years since first menses) |
| Birth control use |
| Drug use (illegal or legal) |
| Body mass index (current, lowest ever, highest ever) |
| History of polyps |
| History of hormonal imbalance |
| History of amenorrhoea |
| History of eating disorders |
| Alcohol consumption by patient, sisters, mother, grandmothers |
| Details of mother's pregnancy with patient: any drugs taken, smoking, alcohol, stress levels, exposure to plastics (i.e. Tupperware), composition of diet (see below) |
| Sleep patterns: number of hours a night, continuous/overall |
| Diet: meat, organic produce, vegetables, vitamin or other supplement consumption, dairy (full fat or reduced fat), coffee/tea consumption, folic acid, sugar (complex, artificial, simple), processed food versus home cooked. |
| Exposure to plastics: microwave in plastic, cook with plastic, store food in plastic, plastic water or coffee mugs. |
| Water consumption: amount per day, format: straight from the tap, bottled water (plastic or bottle), filtered (type Britta/Pur) |
| Residence history starting with mother's pregnancy: location/duration |
| Environmental exposure to potential toxins for different regions (extracted from government monitoring databases) |
| Health metrics: autoimmune disease, chronic illness/condition |
| Pelvic surgery history |
| Life time number of pelvic X-rays |
| History of sexually transmitted infections: type/treatment/outcome |
| Reproductive hormone levels: follicle stimulating hormone, anti-Mullerian hormone, estrogen, progesterone |
| Stress |
| Thickness and type of endometrium throughout the menstrual cycle. |
| Age |
| Height |
| Fertility treatment history and details: history of hormone stimulation, brand of drugs used, basal antral follicle count, follicle count after stimulation with different protocols, number/quality/stage of retrieved oocytes/ development profile of embryos resulting in vitro insemination (natural or ICSI), details oflVF procedure (which clinic, doctor/embryologist at clinic, assisted hatching, fresh or thawed oocytes/embryos, embryo transfer (blood on the catheter/squirt detection and direction on ultrasound) |
| Morning sickness during pregnancy |
| Breast size before/during/after pregnancy |
| History of ovarian cysts |
| Twin or sibling from multiple birth (mono-zygotic or di-zygotic) |
| Male factor infertility for reproductive partner: Semen analysis (count, motility,morphology), Vasectomy, male cancer, smoking, alcohol, diet, STIs |

and
c) conducting an association study based on results from steps (a) and (b) using weighted predictor variables to assess infertility and/or egg quality in the post-pubescent mammal.

The method of the invention may further comprise conducting an assay on an abnormal gene product.

In a preferred method of the invention, the variation is selected from the group consisting of: a single nucleotide polymorphism; a deletion; an insertion; a rearrangement; a copy number variation; and a combination thereof.

In other preferred methods of the invention the gene product is a product of a maternal effect gene; optionally wherein the maternal effect gene is a gene from Table 1.

In a preferred method of the invention, the gene product is RNA or protein.

In assays in accordance with the invention, an amount of the gene product is determined and compared to a reference.

Disclosed herein is how one or more factors, such as genetic variations (e.g., mutations, polymorphisms, expression levels) and phenotypic traits or environmental exposures in order to arrive at an assessment of the potential for egg quality (including egg viability) and fertility generally over time.

Certain genetic polymorphisms may give rise to a predisposition to conditions that affect fertility, such as premature ovarian failure or premature decline in ovarian function. Those conditions reduce egg count and/or viability and have a significant affect on fertility. Genetic factors increase the risk of premature depletion of ovarian reserves and may in themselves indicate the need for clinical intervention, such as a decision to harvest eggs for preservation. Moreover, specific combinations of genetic polymorphisms are significant with respect to a patient's fertility status.

Disclosed herein an array of genetic information concerning the status of various maternal effect genes is used in order to assess egg quality, egg viability, and ultimate fertility status. The genetic information may comprise one or more polymorphisms in one or more maternal effect genes, mutations in one or more of those genes, or epigenetic factors affecting the expression of those genes. The molecular consequence of these gene mutations could be alternative splicing, lowered or increased RNA expression, or alterations in protein expression. These alterations could include a different protein product being produced, such as one with reduced or increased activity, a protein that elicits an abnormal immunological reaction that could be damaging to the ovary or egg cells. A disruption in a fertility related protein can also lead to it failing to oligomerize or bind to other proteins in a complex that it required to bind to for normal egg viability. For example, alterations in transcription factor proteins can significantly advance the rate at which eggs are triggered to mature, leading to premature depletion of eggs from the ovary. Also, methylation patterns in fertility gene containing loci may affect these same processes. All of this information is significant in terms of informing a patient of the likelihood of future premature ovarian failure.

In addition to looking exclusively at genomic information, by combining genetic information (e.g., polymorphisms, mutations, etc.) with phenotypic and/or environmental data, methods of the invention provide an additional level of clinical clarity. For example, polymorphisms in genes discussed below may provide information about a disposition toward premature ovarian failure. However, in certain cases, the clinical outcome may not be determinative unless combined with certain phenotypic and/or environmental information. Thus, methods of the invention provide for a combination of genetic predispositional analysis in combination with phenotypic and environmental exposure data in order to assess the potential for egg quality and viability; as well as the likelihood of continued viability as the woman ages.

Disclosed herein is how a genetic predisposition to ovarian failure is determined, and an assessment of the impact of the genetic predisposition is made. If the genetic analysis is indeterminant, then the genetic data are combined with phenotypic and/or environmental data in order to inform the clinical indication. For example, if it is determined that a woman's egg cells produce a protein variant that is known to potentially cause an autoimmune response, that information may be combined with phenotypic data in order to determine whether an autoimmune response leading to premature ovarian failure is likely. If it is determined that an autoimmune response is likely, eggs can be harvested or ovaries can be preserved for later use.

Thus, in certain cases, genetic predisposition may be sufficient to make a diagnosis, but in other cases, the clinical outcome may not be clear based upon genetic analysis alone and the combination of genetic and phenotypic or environmental data must be used in order to assess the likelihood of ovarian failure and egg quality.

Described herein are methods for assessing infertility in a mammal that involve conducting an assay to determine presence or absence of a mutation in a plurality of genes selected from Table 1 in which the presence of a mutation in at least two of these genes is indicative of poor egg quality or infertility. In particular embodiments, the assay is conducted on all of the
genes from Table 1. Mutations detected according to the invention may be any type of genetic mutation. Exemplary mutations include a single nucleotide polymorphism, a deletion, an insertion, an inversion, other rearrangements, a copy number variation, or a combination thereof.

Any sequencing method of detecting genetic mutations is useful with methods of the invention,
and numerous methods are known in the art. Sequencing is used to determine the presence of a mutation in the genes from Table 1. In particularly-preferred embodiments, the sequencing is sequencing-by-synthesis.

Methods of the invention may further involve obtaining a sample from the mammal that includes the plurality of genes from Table 1. The sample may be a human tissue or body fluid. The sample may be collected
at any age before, during, or after puberty. In particular embodiments, the sample is of maternal origin, such as blood or saliva. In particular embodiments, samples are derived from both the male and female partners who are trying to conceive. Methods of the invention may also involve enriching the sample for the plurality of genes from Table 1.

An infertility-associated phenotypic trait or environmental
exposure is used in combination with genomic results in order to assess egg quality or fertility generally. Exemplary "phenotypic traits" include age, cholesterol levels, body mass index, and combinations thereof. Exemplary "environmental exposures" include smoking, alcohol intake, composition of diet, residence history, or combinations thereof.

There is also described methods for assessing infertility in a mammal that involve a) conducting an assay on at least one infertility-associated biomarker, b) analyzing at least one infertility-associated phenotypic trait or environmental exposure of the mammal, and c) correlating results from steps (a) and (b), thereby assessing infertility in the mammal. Exemplary phenotypic traits or environmental exposures include age, smoking, body mass index, and combinations thereof.

A biomarker generally refers to a molecule that acts as an indicator of a biological state. A biomarker may be a gene; e.g. a maternal effect gene, more particularly a gene
from Table 1. An assay known in the art may be used to analyze the gene.

The assay may include sequencing at least a portion of the gene to determine presence or absence of a mutation that is associated with infertility.

A further biomarker, which is an abnormal gene product, may be analysed.
The gene product may be a product of a maternal effect gene, and in more particular embodiments, the maternal effect gene is a gene from Table 1. The gene product may be RNA or protein. Any assay known in the art may be used to analyze the gene product.
The assay may involve determining an amount of the gene product and comparing the determined amount to a reference.

There is described methods involving obtaining a sample from the mammal that includes the infertility-associated biomarker. The sample may be a human tissue or body fluid. In particular embodiments, the sample is maternal blood or maternal saliva. There is also described the method of enriching the sample for the infertility-associated biomarker.

There is described an array including a substrate, and a plurality of oligonucleotides attached to the substrate at discrete addressable positions, in which at least one of the oligonucleotides hybridizes to a portion of a gene from Table 1 that includes an infertility associated mutation.

### Brief Description of the Drawings

Fig. 1 depicts three maternal effect genes (P ADI6, NLRP5, and OOEP; see Table 1) that are highly conserved, both at the protein, genetic, and gene expression level in humans (H.s.) and mice (M.m.). Fig. 1 also depicts putative fertility-related genes that are clustered with each of the three maternal effect genes.
Fig. 2 depicts important mammalian egg structures that infertility-associated genes localize to and regulate.
Figs. 3-7 depicts the gene expression profile of a number of maternal effect genes in mice and humans.

### Detailed Description

Genetic variation is correlated with egg quality and fertility and is used to assess infertility in a patient and to select appropriate therapies and methods including *in vitro* fertilization and egg preservation. Methods of the invention analyze infertility-associated biomarkers such as genes or gene products, and use results of that analysis to evaluate and/or quantify factors determinative of fertility in an individual, as defined in claim 1.

### Samples

There is described the method of obtaining a sample, e.g., a tissue or body fluid, that is suspected to include an infertility-associated gene or gene product, for example, at least one gene from Table 1 or a gene product (e.g., RNA or protein) of at least one gene from table 1. A sample may be collected in any clinically acceptable manner. A tissue is a mass of connected cells and/or extracellular matrix material, e.g. skin tissue, endometrial tissue, nasal passage tissue, CNS tissue, neural tissue, eye tissue, liver tissue, kidney tissue, placental tissue, mammary gland tissue, placental tissue, gastrointestinal tissue, musculoskeletal tissue, genitourinary tissue, bone marrow, and the like, derived from, for example, a human or other mammal and includes the connecting material and the liquid material in association with the cells and/or tissues. A body fluid is a liquid material derived from, for example, a human or other mammal. Such body fluids include, but are not limited to, mucous, blood, plasma, serum, serum derivatives, bile, blood, maternal blood, phlegm, saliva, sweat, amniotic fluid, menstrual fluid, mammary fluid, follicular fluid of the ovary, fallopian tube fluid, peritoneal fluid, urine, and cerebrospinal fluid (CSF), such as lumbar or ventricular CSF. A sample may also be a fine needle aspirate or biopsied tissue. A sample also may be media containing cells or biological material. Infertility-associated genes or gene products may be found in reproductive cells or tissues, such as gametic cells, gonadal tissue, placenta and non-human embryos. The sample may be drawn from maternal blood or saliva.

Nucleic acid can be extracted from the sample according to methods known in the art. See for example, Maniatis, et al., Molecular Cloning: A Laboratory Manual, Cold Spring Harbor, N.Y., pp. 280-281, 1982. In certain embodiments, a genomic sample is collected from a subject followed by enrichment for genes or gene fragments of interest, for example by hybridization to a nucleotide array comprising fertility-related genes or gene fragments of interest. The sample may be enriched for genes of interest (e.g., infertility-associated genes) using methods known in the art, such as hybrid capture. See for examples, Lapidus (U.S. patent number 7,666,593).

RNA may be isolated from eukaryotic cells by procedures that involve lysis of the cells and denaturation of the proteins contained therein. Tissue of interest includes gametic cells, gonadal tissue, endometrial tissue, placenta, and non-human embryos. RNA may be isolated from fluids of interest by procedures that involve denaturation of the proteins contained therein. Fluids of interest include menstrual fluid, mammary fluid, follicular fluid of the ovary, peritoneal fluid, or culture medium. Additional steps may be employed to remove DNA. Cell lysis may be accomplished with a nonionic detergent, followed by micro centrifugation to remove the nuclei and hence the bulk of the cellular DNA. RNA is extracted from cells of the various types of interest using guanidinium thiocyanate lysis followed by CsCl centrifugation to separate the RNA from DNA (Chirgwin et al., Biochemistry 18:5294-5299 (1979)). Poly(A)+ RNA is selected by selection with oligo-dT cellulose (see Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, separation of RNA from DNA can be accomplished by organic extraction, for example, with hot phenol or phenol/ chloroform/isoamyl alcohol. If desired, RNase inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or Sephadex.TM. (see Ausubel et al., CURRENT PROTOCOLS IN MOLECULAR BIOLOGY, vol. 2, Current Protocols Publishing, New York (1994). Once bound, poly(A)+ mRNA is eluted from the affinity column using 2 mM EDTA/0.1% SDS.

### Biomarkers

A biomarker generally refers to a molecule that may act as an indicator of a biological state. Biomarkers may be any marker that is associated with infertility. Exemplary biomarkers include genes and gene products (e.g., RNA and protein). The biomarker may be an infertility-associated gene. An infertility-associated gene is any gene in which variation is associated with a change in fertility. Examples of changes in fertility include, but are not limited to, the following: a homozygous knockout of an infertility-associated gene leads to a complete loss of fertility; a homozygous knockout of an infertility-associated gene is incompletely penetrant and leads to reduction in fertility that varies from individual to individual; a heterozygous knockout is completely recessive, having no effect on fertility; and the infertility-associated gene is X-linked, such that a potential defect in fertility depends on whether a non-functional allele of the gene is located on an inactive X chromosome (Barr body) or on an expressed X chromosome.

According to the invention, the infertility-associated gene is a maternal effect gene, selected from Table 1. Maternal effects genes are genes that have been found to encode key structures and functions in mammalian oocytes (Yurttas et al., Reproduction 139:809-823,2010). Maternal effect genes are described, for example in, Christians et al. (Mol Cell Bioi 17:778-88, 1997); Christians et al., Nature 407:693-694, 2000); Xiao et al. (EMBO J 18:5943-5952, 1999); Tong et al. (Endocrinology 145:1427-1434,2004); Tong et al. (Nat Genet 26:267-268, 2000); Tong et al. (Endocrinology, 140:3720-3726, 1999); Tong et al. (Hum Reprod 17:903-911,2002); Ohsugi et al. (Development 135:259-269,2008); Borowczyk et al. (Proc Natl Acad Sci USA., 2009); and Wu (Hum Reprod 24:415-424,2009).

| **Table 1 - Human Fertility-Related Genes (OMIM ref # / Alternative Designations** | | |
|---|---|---|
| PADI6 | Pla2g4c | PROKI |
| (610363) | (603602) | (606233) |
| NPM2 | TACC3 | PROKRI |
| (608073) | (605303) | (607122) |
| NLRP5 | C60RF221 | PROKR2 |
| (609658 / MATER) | (611687 / FILlA) | (607123) |
| SMARCA4 | TLE6 | DPPA5 |
| (607123/ Brgl) | (612399) | (611111) |
| OOEP | AURKB | ACTL6A |
| (611689/ FlopedlMOEP19) | (604970) | (604958) |
| HSFI | AURKA | CARMI |
| (140580) | (603072) | (603934) |
| ZARI | DDX20 | SPIN |
| (607520) | (606168) | (609936) |
| DPPA3 | FIGLA | OAS1 |
| (608408 / Stella) | (608697) | (164350) |
| DNMT1 | ZP1 | VIL2 |
| (126375) | (195000) | (123900) |
| CTCF | ZP2 | NLRP14 |
| (604167) | (182888) | (609665) |
| PMS2 | ZP3 | CD55 |
| (600259) | (182889) | (125240) |
| BNC1 | IL-10 | IFITM1 |
| (601930) | (124092) | (604456 / Fragilis) |
| BUB1 | KDM1B | GDF3 |
| (602452) | (613081) | (606522) |
| GDF1 | REX1 | CDX2 |
| (602880) | (609614) | (600297) |
| BMP4 | KLF4 | EZH2 |
| (112262) | (602253) | (601573) |
| SUZ12 | SOX17 | MYC |
| (606245) | (610928) | (190080) |
| P53 | DDX43 | C6ORF221 |
| (191170) | (606286) | (611687) |
| BRCA1 | EEF1A1 | BRCA2 |
| (113705) | (130590) | (600185) |
| FMR1 | DIAPH2 | HS6ST1 |
| (309550) | (300108) | (604846) |
| GPC3 | FOXL2 | GALT |
| (300037) | (605597) | (606999) |
| FMR2 | BMP15 | FSHR |
| (300806) | (300247) | (136435) |
| LHCGR | FSHB | UBE3A |
| (152790) | (136530) | (601623) |
| LHB | INHA | AIRE |
| (152780) | (147380) | (607358) |
| EIF2B2 | EIF2B4 | EIF2B5 |
| (606454) | (606687) | (603945) |
| NOG | BMP7 | POLG |
| (602991) | (112267) | (174763) |
| USP9X | XPNPEP2 | ZFX |
| (300072) | (300145) | (314980) |
| CENPI | MAD1L1 | CENPF |
| (300065) | (602686) | (600236) |
| MAD1L2 | XIST | CDX4 |
| (601467) | (314670) | (300025) |
| WT1 | ATM | ATR |
| (607102) | (607585) | (601215) |
| GDF9 | NOBOX | RFPL4A |
| (601918) | (610934) | (612601) |
| GREM2 | FOXE1 | BAX |
| (608832) | (602617) | (600040) |
| AHR | | |
| (600253) | | |

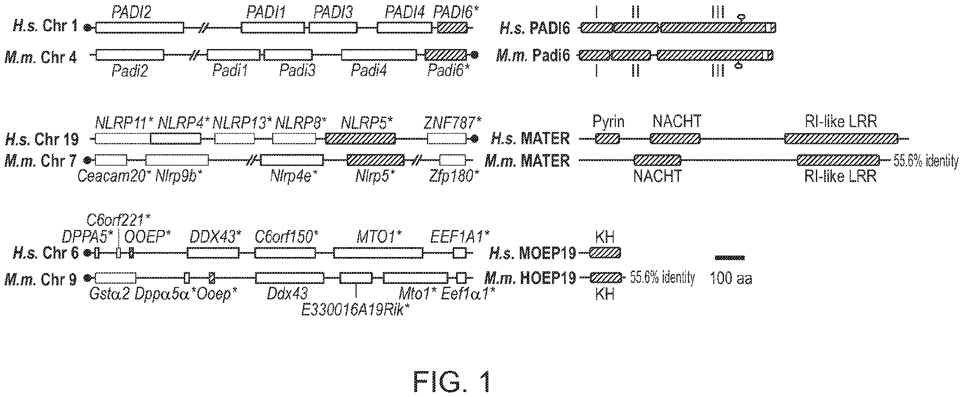

Exemplary genes to be used according to the invention are depicted in Fig. 1. Specifically, Fig. 1 depicts three maternal effect genes (PADI6, NLRP5, and OOEP; see Table 1) that are highly conserved, both at the protein level and genetic level in humans (H.s.) and mice (M.m.). Fig. 1 also depicts putative fertility-related genes that are clustered with each of the three maternal effect genes. These genes are located in genetic loci that are syntenic or highly conserved, and in most cases are observed to be expressed in murine and/or human eggs. Accordingly, fertility-related genes clustered with PADI6, NLRP5, OOEP, and other maternal effect genes including but not limited to those depicted in Fig. 1, can also be selected for use according to the invention.

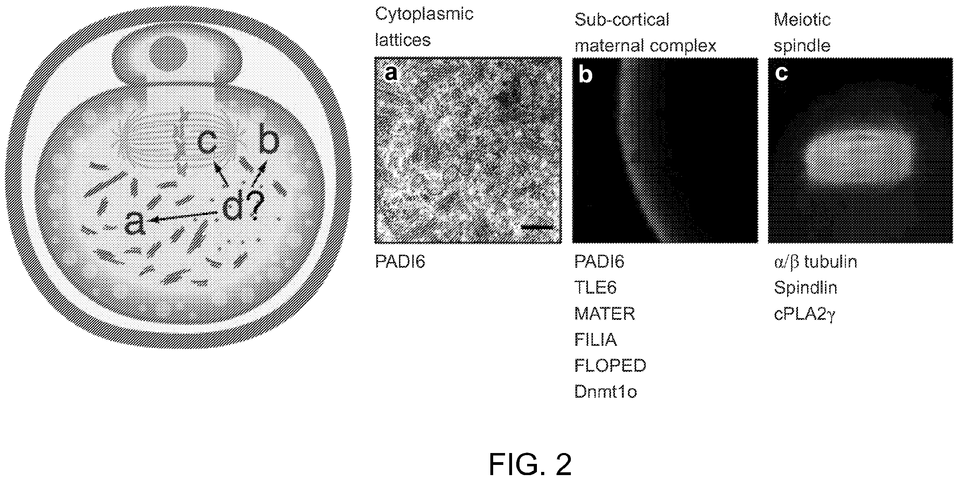

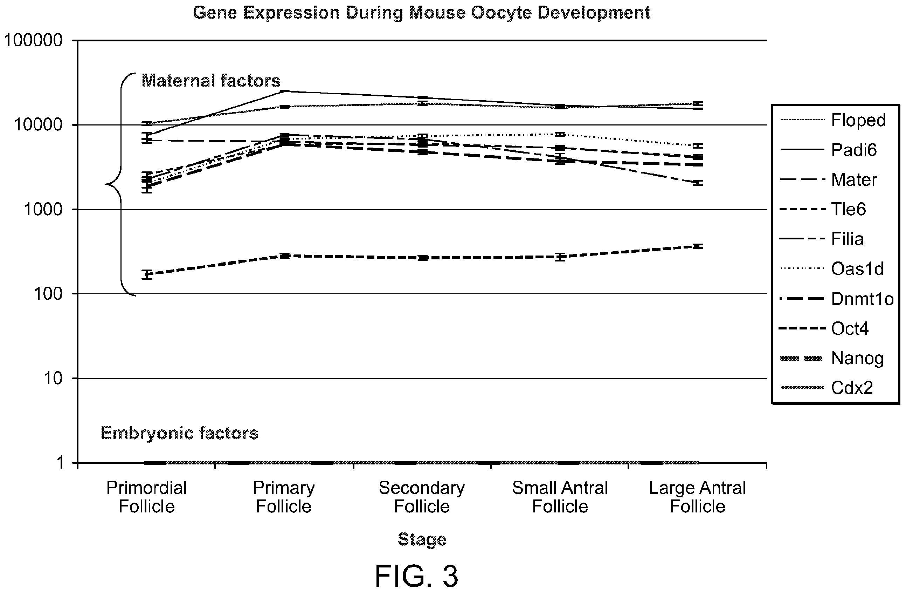

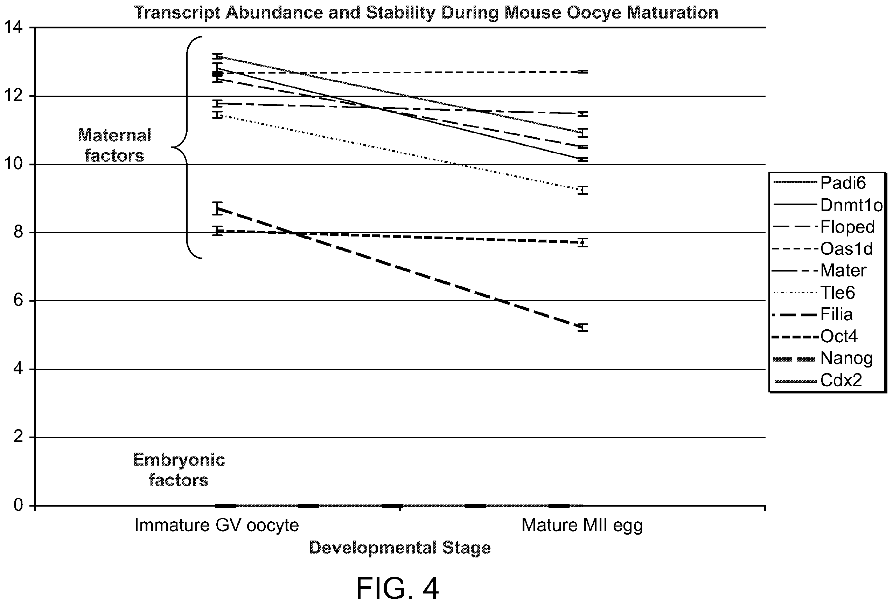

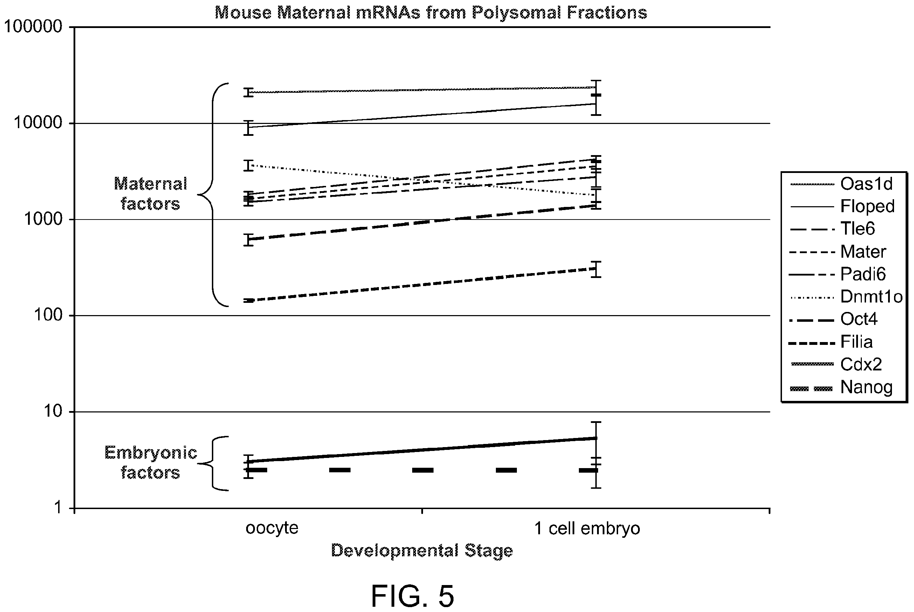

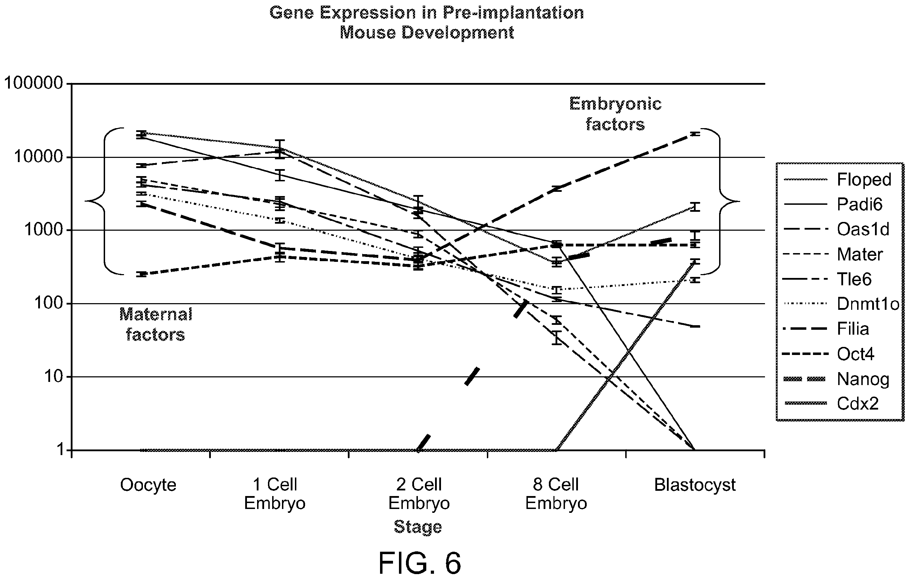

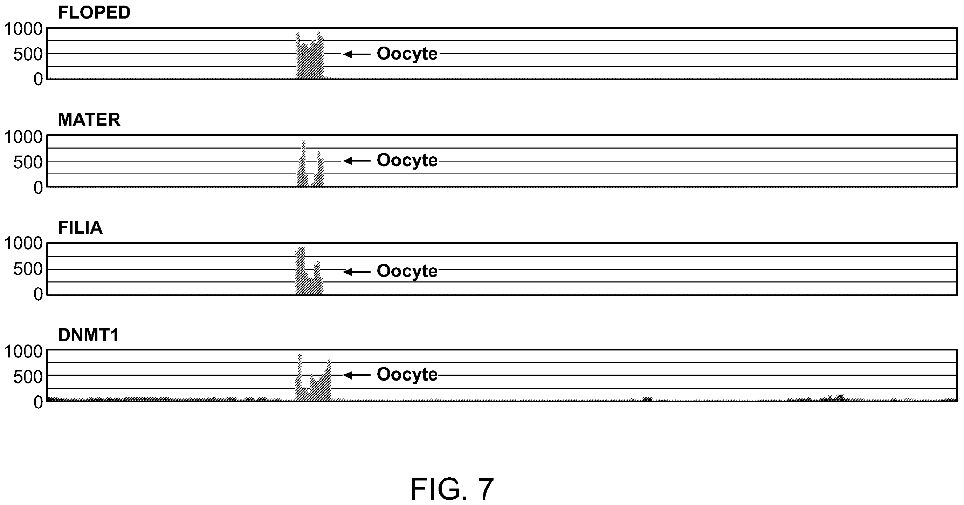

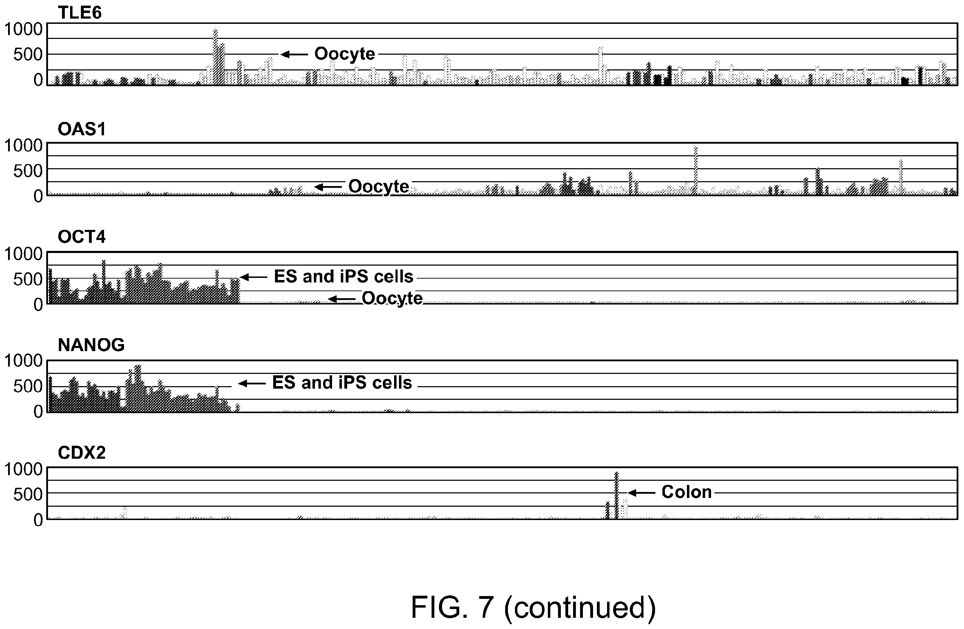

The molecular products of the genes in Table 1 are involved in different aspects of oocyte and embryo physiology from transcription and chromosome remodeling to RNA processing and binding. See Figs. 3-7. Fig. 2 depicts important mammalian egg structures: the Cytoplasmic Lattices, the Subcortical Maternal Complex (SCMC), and the Meiotic Spindle, that infertility-associated genes localize to and regulate. Fig. 3 depicts the abundant gene expression profile throughout egg development in mice of the maternal effect genes (MEG) associated with the SCMC. The relatively lower gene expression pattern of three genes (*Oct4, Cdx2, Nanog*) not associated with this complex are shown as a control. Fig. 4 depicts the relative decline in expression levels of the maternal effect genes associated with the SCMC during mouse oocyte maturation. Decline in MEG levels is commonly observed during oocyte maturation. Fig. 5 depicts the enrichment of MEGs associated with the SCMC in the polysomal fraction during the maternal to zygotic transition in mouse. Presence in this fraction is evidence that these mRNAs are being translated into protein. Fig. 6 depicts the dynamic RNA levels of MEGs associated with the SCMC (oocyte factors) relative to embryo factors (*Oct4, Cdx2, Nanog*) during the course of pre-implantation development in mice. In general, the levels of maternally inherited transcripts decline until the embryonic genome is fully activated. Fig. 7 depicts the tissue specific expression of human maternal effect genes.

**Peptidylarginine deiminase 6 (PADI6)** PADI6 was originally cloned from a 2D egg proteome gel based on its relative abundance, and PADI6 expression appears to be almost entirely limited to the oocyte and pre-implantation embryo. PADI6 is first being expressed in primordial oocyte follicles and persists, at the protein level, throughout pre-implantation development to the blastocyst stage (Wright et al., Dev Biol, 256:73-88, 2003). Inactivation of PADI6 leads to female infertility in mice, with the *Padi6-null* developmental arrest occurring at the two-cell stage.

**Nucleoplasmin 2 (NPM2)** Nucleoplasmin is a mammalian maternal effect gene, which is thought to be phosphorylated during oocyte maturation. NPM2 exhibits a phosphate sensitive increase in mass during oocyte maturation. Increased phosphorylation is retained through the pronuclear stage of development. NPM2 then becomes dephosphorylated at the two cell stage and remains in this form throughout the rest of pre-implantation development. Further, its expression pattern appears to be restricted to oocytes and early embryos. Immunofluorescence analysis of NPM2 localization shows that NPM2 primarily localizes to the nucleus in oocytes and early embryos. In mice, maternally-derived NPM2 is required for
female fertility.

**Maternal antigen the embryos require (MATER** / **NLRP5)** MATER is another highly abundant mouse oocyte protein that is essential for embryonic development beyond the two-cell stage. MATER was originally identified as an oocyte-specific antigen in a mouse model of autoimmune premature ovarian failure (Tong et al., Endocrinology, 140:3720-3726, 1999). MATER demonstrates a similar expression and subcellular expression profile to PADI6. Like PADI6 null animals, Mater null females exhibit normal oogenesis, ovarian development, oocyte maturation, ovulation and fertilization. However, embryos derived from Mater-null females undergo a developmental block at the two-cell stage and fail to exhibit normal embryonic genome activation (Tong et al., Nat Genet 26:267-268, 2000; and Tong et al. Mamm Genome 11:281-287, 2000b).

**Brahma-related gene 1 (Brg1)** Mammalian SWI/SNF-related chromatin remodeling complexes regulate transcription and are believed to be involved in zygotic genome activation (ZGA). Such complexes are composed of approximately nine subunits, which can be variable depending on cell type and tissue. The BRG1 catalytic subunit exhibits DNA-dependent APTase activity, and the energy derived from ATP hydrolysis alters the conformation and position of nucleosomes. Brg1 is expressed in oocytes and has been shown to be essential in the mouse as null homozygotes do not progress beyond the blastocyst stage.

**Factor located in oocytes permitting embryonic development (Floped /OOEP)** The subcortical maternal complex (SCMC) is a poorly characterized mammalian oocyte structure to which several maternal effect gene products localize (Li et al. Dev Cell 15:416-425, 2008). PADI6, MATER, FILIA, TLE6, and FLOPED have been shown to localize to this complex (Li et al. Dev Cell 15:416-425, 2008; Yurttas et al. Development 135:2627-2636, 2008). This complex is not present in the absence of FLOPED and MATER, and similar to embryos resulting from MATER depleted oocytes, embryos resulting from *Floped-null* oocytes do not progress past the two cell stage of development. FLOPED is a small (19kD) RNA binding protein that has also been characterized under the name of MOEP19 (Herr et al., Dev Biol 314:300-316, 2008).

**FILIA** FILIA is another small RNA-binding domain containing maternally inherited protein. FILIA was identified and named for its interaction with MATER (Ohsugi et al. Development 135:259-269, 2008). Like other components of the SCMC, maternal inheritance of the *Filia* gene product is required for early embryonic development. Loss of FILIA results in a developmental arrest of varying severity with a high incidence of aneuploidy due, in part, to improper chromosome alignment during early cleavage divisions. Also the spindle assembly checkpoint is aberrant in these embryos (Zheng et al. Proc Natl Acad Sci USA 106:7473-7478, 2009).

**Basonuclin (Bnc1)** Basonuclin is a zinc finger transcription factor found in keratinocytes and germ cells (male and female) that regulates rDNA (pol I) and mRNA (pol II) synthesis. Depending on the amount by which expression is reduced in oocytes, embryos may not develop beyond the 8 cell stage. In *Bsn1* depleted mice, a normal number of oocytes are ovulated even though oocyte development is perturbed, but many of these oocytes cannot go on to yield viable offspring.

**Zygote Arrest 1 (Zar1)** Zar1 is an oocyte-specific maternal effect gene that functions at the oocyte to embryo transition. High levels of Zarl expression are observed in the cytoplasm of murine oocytes, and homozygous-null females are infertile: growing oocytes from Zar1-/- females did not progress past the two-cell stage.

**Basonuclin (Bnc1)** Basonuclin is a zinc finger transcription factor found in keratinocytes and germ cells (male and female) that regulates rDNA (pol I) and mRNA (pol II) synthesis.. Depending on the amount by which expression is reduced in oocytes, embryos may not develop beyond the 8 cell stage. In bsn1 depleted mice a normal number of oocytes are ovulated even though oocyte development is perturbed, but many of these oocytes cannot go on to yield viable offspring.

**Cytosolic phospholipase A2**γ **(Pla2g4c** / **cPLA2γ)** Under normal conditions, cPLA2γ expression is restricted to oocytes and early embryos in mice. At the subcellular level, cPLA2γ mainly localizes to the cortical regions, nucleoplasm, and multivesicular aggregates of oocytes. It is also worth noting that while cPLA2γ expression does appear to be mainly limited to oocytes and pre-implantation embryos in healthy mice, expression is considerably up-regulated within the intestinal epithelium of mice infected with *Trichinella spiralis.* This suggests that cPLA2γ may also play a role in the inflammatory response. The human cPLA2γ orthologue differs in that rather than being abundantly expressed in the ovary, it is abundantly expressed in the heart and skeletal muscle. Also, the human protein contains a lipase consensus sequence but lacks a calcium binding domain found in other PLA2 enzymes. Accordingly, another cytosolic phospholipase may be a better candidate.

**Transforming, Acidic Coiled-Coil Containing Protein 3 (TACC3)** In mice, Maskin/TACC3 is required for microtubule anchoring at the centrosome and for spindle assembly and cell survival.

The gene may be a gene that is expressed in an oocyte. Exemplary genes include *CTCF* (Wan et al., Development 135:2729-2738, 2009), *Zfp57* (Li et al. Dev Cell 15:416-425, 2008), *Oct4* (Zuccotti et al., Reprod Biomed Online 19 Suppl 3:57-62, 2009a; Zuccotti et al., BMC Dev Biol 8:97, 2008; and Zuccotti et al. Hum Reprod 24:2225-2237, 2009b), *SEBOX* (Kim et al., Biol Reprod 79:1192-201, 2008), *HDAC1* (Ma et al. Dev Biol 319:110-120, 2008), and *Pms2* (Gurtu et al. Genetics 160:271-277, 2002).

The gene may be a gene that is involved in DNA repair pathways, including but not limited to, *Mlh1, Pms1* and *Pms2.* The gene may be a *BRCA* gene.

The biomarker may be a gene product (e.g., RNA or protein) of an
infertility-associated gene. The gene product may be a gene product of a maternal effect gene. The gene product may be a product of a gene from Table 1. The gene product may be a product of a gene that is expressed in an oocyte, such as a product of *CTCF, Zfp57, Oct4, SEBOX, HDAC1*, or *Pms2.* The gene product is a product of a gene that is involved in DNA repair pathways, such as a product of *Mlh1, Pms1,* or *Pms2.* The gene product may be a product of a *BRCA.*

The biomarker may be an epigenetic factor, such as methylation patterns (e.g., CpG islands), histone proteins, acetylation, ubiquitination, phosphorylation, or others.

### Assays

Described herein is conducting an assay that detects either a mutations in an infertility-associated gene or abnormal expression (over or under) of an infertility-associated gene product. The assay may be conducted on genes from Table 1 or
products of genes from Table 1. Detailed descriptions of conventional methods, such as those employed to make and use nucleic acid arrays, amplification primers, hybridization probes, and the like can be found in standard laboratory manuals such as: Genome Analysis: A Laboratory Manual Series (Vols. I-IV), Cold Spring Harbor Laboratory Press; PCR Primer: A Laboratory Manual, Cold Spring Harbor Laboratory Press; and Sambrook, J et al., (2001) Molecular Cloning: A Laboratory Manual, 2nd ed. (Vols. 1-3), Cold Spring Harbor Laboratory Press. Custom nucleic acid arrays are commercially available from, *e*.*g*., Affymetrix (Santa Clara, CA), Applied Biosystems (Foster City, CA), and Agilent Technologies (Santa Clara, CA).

Methods of detecting mutations in genes are known in the art. In accordance with the invention the assay is conducted on at least six genes from Table 1(e.g., all of the genes from Table 1), and a mutation in at least six of the genes from Table 1 indicates infertility; a mutation in at least seven of the genes from Table 1 indicates infertility; a mutation in at least eight of the genes from Table 1 indicates infertility; a mutation in at least nine of the genes from Table 1 indicates infertility; a mutation in at least 10 of the genes from Table 1 indicates infertility; a mutation in at least 15 of the genes from Table 1 indicates infertility; or a mutation in all of the genes from Table 1 indicates infertility.

In certain embodiments, a known single nucleotide polymorphism at a particular position can be detected by a single base extension for a primer that binds to the sample DNA adjacent to that position. See for example Shuber et al. (U.S. patent number 6,566,101). In other embodiments, a hybridization probe might be employed that overlaps the SNP of interest and selectively hybridizes to sample nucleic acids containing a particular nucleotide at that position. See for example Shuber et al. (U.S. patent number 6,214,558 and 6,300,077).

In particular embodiments, nucleic acids are sequenced in order to detect variants (i.e., mutations) in the nucleic acid compared to wild-type and/or non-mutated forms of the sequence. The nucleic acid can include a plurality of nucleic acids derived from a plurality of genetic elements. Methods of detecting sequence variants are known in the art, and sequence variants can be detected by any sequencing method known in the art e.g., ensemble sequencing or single molecule sequencing.

One conventional method to perform sequencing is by chain termination and gel separation, as described by Sanger et al., Proc Nati. Acad. Sci. USA, 74(12): 5463 67 (1977). Another conventional sequencing method involves chemical degradation of nucleic acid fragments. See, Maxam et al., Proc. Nati. Acad. Sci., 74: 560564 (1977). Finally, methods have been developed based upon sequencing by hybridization. See, e.g., Harris et al., (U.S. patent application number 2009/0156412).

In certain embodiments, sequencing is performed by the Sanger sequencing technique. Classical Sanger sequencing involves a single-stranded DNA template, a DNA primer, a DNA polymerase, radioactively or fluorescently labeled nucleotides, and modified nucleotides that terminate DNA strand elongation. If the label is not attached to the dideoxynucleotide terminator (e.g., labeled primer), or is a monochromatic label (e.g., radioisotope), then the DNA sample is divided into four separate sequencing reactions, containing four standard deoxynucleotides (dATP, dGTP, dCTP and dTTP) and the DNA polymerase. To each reaction is added only one of the four dideoxynucleotides (ddATP, ddGTP, ddCTP, or ddTTP). These dideoxynucleotides are the chain-terminating nucleotides, lacking a 3'-OH group required for the formation of a phosphodiester bond between two nucleotides during DNA strand elongation. If each of the dideoxynucleotides carries a different label, however, (e.g., 4 different fluorescent dyes), then all the sequencing reactions can be carried out together without the need for separate reactions.

Incorporation of a dideoxynucleotide into the nascent, i.e., elongating, DNA strand terminates DNA strand extension, resulting in a nested set of DNA fragments of varying length. Newly synthesized and labeled DNA fragments are denatured, and separated by size using gel electrophoresis on a denaturing polyacrylamide-urea gel capable of resolving single-base differences in chain length. If each of the four DNA synthesis reactions was labeled with the same, monochromatic label (e.g., radioisotope), then they are separated in one of four individual, adjacent lanes in the gel, in which each lane in the gel is designated according to the dideoxynucleotide used in the respective reaction, i.e., gel lanes A, T, G, C. If four different labels were utilized, then the reactions can be combined in a single lane on the gel. DNA bands are then visualized by autoradiography or fluorescence, and the DNA sequence can be directly read from the X-ray film or gel image.

The terminal nucleotide base is identified according to the dideoxynucleotide that was added in the reaction resulting in that band or its corresponding direct label. The relative positions of the different bands in the gel are then used to read (from shortest to longest) the DNA sequence as indicated. The Sanger sequencing process can be automated using a DNA sequencer, such as those commercially available from PerkinElmer, Beckman Coulter, Life Technologies, and others.

In other embodiments, sequencing of the nucleic acid is accomplished by a single-molecule sequencing by synthesis technique. Single molecule sequencing is shown for example in Lapidus et al. (US. patent number 7,169,560), Quake et al. (US. patent number 6,818,395), Harris (US. patent number 7,282,337), Quake et al. (U.S. patent application number 2002/0164629), and Braslavsky, et al., PNAS (USA), 100: 3960-3964 (2003). Briefly, a single-stranded nucleic acid (e.g., DNA or cDNA) is hybridized to oligonucleotides attached to a surface of a flow cell. The oligonucleotides may be covalently attached to the surface or various attachments other than covalent linking as known to those of ordinary skill in the art may be employed. Moreover, the attachment may be indirect, e.g., via a polymerase directly or indirectly attached to the surface. The surface may be planar or otherwise, and/or may be porous or non-porous, or any other type of surface known to those of ordinary skill to be suitable for attachment. The nucleic acid is then sequenced by imaging the polymerase-mediated addition of fluorescently-labelled nucleotides incorporated into the growing strand surface oligonucleotide, at single molecule resolution.

Other single molecule sequencing techniques involve detection of pyrophosphate as it is cleaved from incorporation of a single nucleotide into a nascent strand of DNA, as is shown in Rothberg et al. (U.S. patent numbers 7,335,762, 7,264,929, 7,244,559, and 7,211,390) and Leamon et al. (U.S. patent number 7,323,305).

If the nucleic acid from the sample is degraded or only a minimal amount of nucleic acid can be obtained from the sample, PCR can be performed on the nucleic acid in order to obtain a sufficient amount of nucleic acid for sequencing (See e.g., Mullis et al. US. patent number 4,683,195).

Methods of detecting levels of gene products (e.g., RNA or protein) are known in the art. Commonly used methods known in the art for the quantification of mRNA expression in a sample include northern blotting and in situ hybridization (Parker & Barnes, Methods in Molecular Biology 106:247283 (1999),); RNAse protection assays (Hod, Biotechniques 13:852854 (1992),); and PCR-based methods, such as reverse transcription polymerase chain reaction (RT-PCR) (Weis et al., Trends in Genetics 8:263 264 (1992)). Alternatively, antibodies may be employed that can recognize specific duplexes, including RNA duplexes, DNA-RNA hybrid duplexes, or DNA-protein duplexes. Other methods known in the art for measuring gene expression (e.g., RNA or protein amounts) are shown in Yeatman et al. (U.S. patent application number 2006/0195269).

A differentially expressed gene or differential gene expression refer to a gene whose expression is activated to a higher or lower level in a subject suffering from a disorder, such as infertility, relative to its expression in a normal or control subject. The terms also include genes whose expression is activated to a higher or lower level at different stages of the same disorder. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example.

Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disorder, such as infertility, or between various stages of the same disorder. Differential expression includes both quantitative, as well as qualitative, differences in the temporal or cellular expression pattern in a gene or its expression products. Differential gene expression (increases and decreases in expression) is based upon percent or fold changes over expression in normal cells. Increases may be of 1, 5, 10, 20, 30, 40, 50, 60, 70, 80, 90, 100, 120, 140, 160, 180, or 200% relative to expression levels in normal cells. Alternatively, fold increases may be of 1, 1.5, 2, 2.5, 3, 3.5, 4, 4.5, 5, 5.5, 6, 6.5, 7, 7.5, 8, 8.5, 9, 9.5, or 10 fold over expression levels in normal cells. Decreases may be of 1, 5, 10, 20, 30, 40, 50, 55, 60, 65, 70, 75, 80, 82, 84, 86, 88, 90, 92, 94, 96, 98, 99 or 100% relative to expression levels in normal cells.

In certain embodiments, reverse transcriptase PCR (RT-PCR) is used to measure gene expression. RT-PCR is a quantitative method that can be used to compare mRNA levels in different sample populations to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andres et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MASTERPURE™ Complete DNA and RNA Purification Kit (EPICENTRE®, Madison, Wis.), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

The first step in gene expression profiling by RT-PCR is the reverse transcription of the RNA template into eDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp® RNA PCR kit (Perkin Elmer, Calif., USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, Calif., USA), or Lightcycler® (Roche Molecular Biochemicals, Mannheim, Germany). In certain embodiments, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Cₜ).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin. For performing analysis on pre-implantation embryos and oocytes, Chuk is a gene that is used for normalization.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, in which internal competitor for each target sequence is used for normalization, and with quantitative comparative PCR using a normalization gene contained within the sample, or a housekeeping gene for RT-PCR. For further details see, e.g. Held et al., Genome Research 6:986 994 (1996),

In another embodiment, a MassARRAY®-based gene expression profiling method is used to measure gene expression. In the MassARRAY®-based gene expression profiling method, developed by Sequenom, Inc. (San Diego, Calif.) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059 3064 (2003).

Further PCR-based techniques include, for example, differential display (Liang and Pardee, Science 257:967 971 (1992)); amplified fragment length polymorphism (iAFLP) (Kawamoto et al., Genome Res. 12:1305 1312 (1999)); BeadArray™ technology (Illumina, San Diego, Calif.; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex®100 LabMAP system and multiple color-coded microspheres (Luminex Corp., Austin, Tex.) in a rapid assay for gene expression (Yang et al., Genome Res. 11:1888 1898 (2001)); and high coverage expression profiling (HiCEP) analysis (Fukumura et al., Nucl. Acids. Res. 31(16) e94 (2003)).

In certain embodiments, differential gene expression can also be identified, or confirmed using a microarray technique. In this method, polynucleotide sequences of interest (including cDNAs and oligonucleotides) are plated, or arrayed, on a microchip substrate. The arrayed sequences are then hybridized with specific DNA probes from cells or tissues of interest. Methods for making microarrays and determining gene product expression (e.g., RNA or protein) are shown in Yeatman et al. (U.S. patent application number 2006/0195269).

In a specific embodiment of the micro array technique, PCR amplified inserts of cDNA clones are applied to a substrate in a dense array, for example, at least 10,000 nucleotide sequences are applied to the substrate. The micro arrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pair-wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106 149 (1996)). Microarray analysis can be performed by commercially available equipment, following manufacturer's protocols, such as by using the Affymetrix GenChip technology, or Incyte's micro array technology.

Alternatively, protein levels can be determined by constructing an antibody microarray in which binding sites comprise immobilized, preferably monoclonal, antibodies specific to a plurality of protein species encoded by the cell genome. Preferably, antibodies are present for a substantial fraction of the proteins of interest. Methods for making monoclonal antibodies are well known (see, e.g., Harlow and Lane, 1988, ANTIBODIES: A LABORATORY MANUAL, Cold Spring Harbor, N.Y.). In one embodiment, monoclonal antibodies are raised against synthetic peptide fragments designed based on genomic sequence of the cell. With such an antibody array, proteins from the cell are contacted to the array, and their binding is assayed with assays known in the art. Generally, the expression, and the level of expression, of proteins of diagnostic or prognostic interest can be detected through immunohistochemical staining of tissue slices or sections.

Finally, levels of transcripts of marker genes in a number of tissue specimens may be characterized using a "tissue array" (Kononen et al., Nat. Med 4(7):844-7 (1998). In a tissue array, multiple tissue samples are assessed on the same microarray. The arrays allow in situ detection of RNA and protein levels; consecutive sections allow the analysis of multiple samples simultaneously.

In other embodiments, Serial Analysis of Gene Expression (SAGE) is used to measure gene expression. Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484 487 (1995); and Velculescu et al., Cell 88:243 51 (1997).

In other embodiments Massively Parallel Signature Sequencing (MPSS) is used to measure gene expression. This method, described by Brenner et al., Nature Biotechnology 18:630 634 (2000), is a sequencing approach that combines non-gel-based signature sequencing with in vitro cloning of millions of templates on separate 5 µm diameter microbeads. First, a microbead library of DNA templates is constructed by in vitro cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

Immunohistochemistry methods are also suitable for detecting the expression levels of the gene products of the present invention. Thus, antibodies (monoclonal or polyclonal) or antisera, such as polyclonal antisera, specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

In certain embodiments, a proteomics approach is used to measure gene expression. A proteome refers to the totality of the proteins present in a sample (e.g. tissue, organism, or cell culture) at a certain point of time. Proteomics includes, among other things, study of the global changes of protein expression in a sample (also referred to as expression proteomics). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

In some embodiments, mass spectrometry (MS) analysis can be used alone or in combination with other methods (e.g., immunoassays or RNA measuring assays) to determine the presence and/or quantity of the one or more biomarkers disclosed herein in a biological sample. In some embodiments, the MS analysis includes matrix-assisted laser desorption/ionization (MALDI) time-of-flight (TOF) MS analysis, such as for example directspot MALDI-TOF or liquid chromatography MALDI-TOF mass spectrometry analysis. In some embodiments, the MS analysis comprises electro spray ionization (ESI) MS, such as for example liquid chromatography (LC) ESI-MS. Mass analysis can be accomplished using commerciallyavailable spectrometers. Methods for utilizing MS analysis, including MALDI- TOF MS and ESI-MS, to detect the presence and quantity of biomarker peptides in biological samples are known in the art. See for example U.S. Pat. Nos. 6,925,389; 6,989,100; and 6,890,763 for further guidance.

### Phenotypic traits

Methods of the invention assess infertility by correlating assay results with an analysis of a phenotypic trait or environmental exposure that may be associated with infertility. The phenotypic traits or environmental exposures are shown in Table 2.

| **Table 2 - Phenotypic and environmental variables impacting fertility success** |
|---|
| Cholesterol levels on different days of the menstrual cycle |
| Age of first menses for patient, sisters, mother, grandmothers |
| Age of menopause for sisters, mother, grandmothers |
| Number of previous pregnancies (biochemical/ectopic/clinical/fetal heart beat detected), age at the time, and outcome for patient, sisters, mother, grandmothers |
| Polycystic ovarian syndrom |
| History of hydrosalpinx or tubal occlusion |
| History of endometriosis or painful periods |
| Cancer history/type of cancer/treatment/outcome for patient, sisters, mother, grandmothers |
| Age that sexual activity began, current level of sexual activity |
| Smoking history for patient, sisters, mother, grandmothers |
| Travel schedule/number of flying hours a year/time difference changes of more than 3 hours (jetlag) |
| Nature of periods (length of menses, length of cycle) |
| Biological age (number of years since first menses) |
| Birth control use |
| Drug use (illegal or legal) |
| Body mass index (current, lowest ever, highest ever) |
| History of polyps |
| History of hormonal imbalance |
| History of amenorrhoea |
| History of eating disorders |
| Alcohol consumption by patient, sisters, mother, grandmothers |
| Details of mother's pregnancy with patient: any drugs taken, smoking, alcohol, stress levels, exposure to plastics (i.e. Tupperware), composition of diet (see below) |
| Sleep patterns: number of hours a night, continuous/overall |
| Diet: meat, organic produce, vegetables, vitamin or other supplement consumption, dairy (full fat or reduced fat), coffee/tea consumption, folic acid, sugar (complex, artificial, simple), processed food versus home cooked. |
| Exposure to plastics: microwave in plastic, cook with plastic, store food in plastic, plastic water or coffee mugs. |
| Water consumption: amount per day, format: straight from the tap, bottled water (plastic or bottle), filtered (type: Britta/Pur) |
| Residence history starting with mother's pregnancy: location/duration |
| Environmental exposure to potential toxins for different regions (extracted from government monitoring databases) |
| Health metrics: autoimmune disease, chronic illness/condition |
| Pelvic surgery history |
| Life time number of pelvic X-rays |
| History of sexually transmitted infections: type/treatment/outcome |
| Reproductive hormone levels: follicle stimulating hormone, anti-Müllerian hormone, estrogen, progesterone |
| Stress |
| Thickness and type of endometrium throughout the menstrual cycle. |
| Age |
| Height |
| Fertility treatment history and details: history of hormone stimulation, brand of drugs used, basal antral follicle count, follicle count after stimulation with different protocols, number/quality/stage of retrieved oocytes/ development profile of embryos resulting from in vitro insemination (natural or ICSI), details of IVF procedure (which clinic, doctor/embryologist at clinic, assisted hatching, fresh or thawed oocytes/embryos, embryo transfer (blood on the catheter/squirt detection and direction on ultrasound) |
| Morning sickness during pregnancy |
| Breast size before/during/after pregnancy |
| History of ovarian cysts |
| Twin or sibling from multiple birth (mono-zygotic or di-zygotic) |
| Male factor infertility for reproductive partner: Semen analysis (count, motility,morphology), Vasectomy, male cancer, smoking, alcohol, diet, STIs |

Association studies can be performed to analyze the effect of genetic mutations or abnormal gene expression on a particular trait being studied. Infertility as a trait may be analyzed as a non-continuous variable in a case-control study that includes as the patients infertile females and as controls fertile females that are age and ethnically matched. Methods including logistic regression analysis and chi square tests may be used to identify an association between genetic mutations or abnormal gene expression and infertility. In addition, when using logistic regression, adjustments for covariates like age, smoking, BMI and other factors that effect infertility, such as those shown in Table 2, may be included in the analysis.

In addition, haplotype effects can be estimated using programs such as Haploscore. Alternatively, programs such as Haploview and Phase can be used to estimate haplotype frequencies and then further analysis such as Chi square test can be performed. Logistic regression analysis may be used to generate an odds ratio and relative risk for each genetic variant or variants.

The association between genetic mutations and/or abnormal gene expression and infertility may be analyzed within cases only or comparing cases and controls using analysis of variance. Such analysis may include, adjustments for covariates like age, smoking, BMI and other factors that effect infertility. In addition, haplotype effects can be estimated using programs such as Haploscore.

Method of logistic regression are described, for example in, Ruczinski (Journal of Computational and Graphical Statistics 12:475-512,2003); Agresti (An Introduction to Categorical Data Analysis, John Wiley & Sons, Inc., 1996, New York, Chapter 8); and Yeatman et al. (U.S. patent application number 2006/0195269).

Other algorithms for analyzing associations are known. For example, the stochastic gradient boosting is used to generate multiple additive regression tree (MART) models to predict a range of outcome probabilities. Each tree is a recursive graph of decisions the possible consequences of which partition patient parameters; each node represents a question (e.g., is the FSH level greater than x?) and the branch taken from that node represents the decision made (e.g. yes or no). The choice of question corresponding to each node is automated. A MART model is the weighted sum of iteratively produced regression trees. At each iteration, a regression tree is fitted according to a criterion in which the samples more involved in the prediction error are given priority. This tree is added to the existing trees, the prediction error is recalculated, and the cycle continues, leading to a progressive refinement of the prediction. The strengths of this method include analysis of many variables without knowledge of their complex interactions beforehand.

A different approach called the generalized linear model, expresses the outcome as a weighted sum of functions of the predictor variables. The weights are calculated based on least squares or Bayesian methods to minimize the prediction error on the training set. A predictor's weight reveals the effect of changing that predictor, while holding the others constant, on the outcome. In cases where one or more predictors are highly correlated, in a phenomenon known as collinearity, the relative values of their weights are less meaningful; steps must be taken to remove that collinearity, such as by excluding the nearly redundant variables from the model. Thus, when properly interpreted, the weights express the relative importance of the predictors. Less general formulations of the generalized linear model include linear regression, multiple regression, and multifactor logistic regression models, and are highly used in the medical community as clinical predictors.

### Microarrays

A microarray is described including a plurality of oligonucleotides attached to a substrate at discrete addressable positions, in which at least one of the oligonucleotides hybridizes to a portion of a gene from Table 1 that includes an infertility-associated mutation.

Methods of constructing microarrays are known in the art. See for example Yeatman et al. (U.S. patent application number 2006/0195269).

Microarrays are prepared by selecting probes that include a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically in vivo, enzymatically in vitro (e.g., by PCR), or non-enzymatically in vitro.

The probe or probes used in the methods of the invention are preferably immobilized to a solid support which may be either porous or non-porous. For example, the probes may be polynucleotide sequences which are attached to a nitrocellulose or nylon membrane or filter covalently at either the 3' or the 5' end of the polynucleotide. Such hybridization probes are well known in the art (see, e.g., Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, the solid support or surface may be a glass or plastic surface. In a particularly preferred embodiment, hybridization levels are measured to micro arrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel.

In preferred embodiments, a microarray comprises a support or surface with an ordered array of binding (e.g., hybridization) sites or "probes" each representing one of the genes described herein, particularly the genes described in Table 1. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). In preferred embodiments, each probe is covalently attached to the solid support at a single site.

Microarrays can be made in a number of ways, of which several are described below. However produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. The micro arrays are preferably small, e.g., between 1 cm² and 25 cm², between 12 cm² and 13 cm², or 3 cm². However, larger arrays are also contemplated and may be preferable, e.g., for use in screening arrays. Preferably, a given binding site or unique set of binding sites in the micro array will specifically bind (e.g., hybridize) to the product of a single gene in a cell (e.g., to a specific mRNA, or to a specific cDNA derived therefrom). However, in general, other related or similar sequences will cross hybridize to a given binding site.

The microarrays described include one or more test probes, each of which has a polynucleotide sequence that is complementary to a subsequence of RNA or DNA to be detected. Preferably, the position of each probe on the solid surface is known. Indeed, the microarrays are preferably positionally addressable arrays. Specifically, each probe of the array is preferably located at a known, predetermined position on the solid support such that the identity (i.e., the sequence) of each probe can be determined from its position on the array (i.e., on the support or surface).

The microarray is an array (i.e., a matrix) in which each position represents one of the biomarkers described herein. For example, each position can contain a DNA or DNA analogue based on genomic DNA to which a particular RNA or cDNA transcribed from that genetic marker can specifically hybridize. The DNA or DNA analogue can be, e.g., a synthetic oligomer or a gene fragment. In one embodiment, probes representing each of the markers is present on the array. In a preferred embodiment, the array comprises probes for each of the genes listed in Table 1.

As noted above, the probe to which a particular polynucleotide molecule specifically hybridizes according to the invention contains a complementary genomic polynucleotide sequence. The probes of the microarray preferably consist of nucleotide sequences of no more than 1,000 nucleotides. In some embodiments, the probes of the array consist of nucleotide sequences of 10 to 1,000 nucleotides. In a preferred embodiment, the nucleotide sequences of the probes are in the range of 10-200 nucleotides in length and are genomic sequences of a species of organism, such that a plurality of different probes is present, with sequences complementary and thus capable of hybridizing to the genome of such a species of organism, sequentially tiled across all or a portion of such genome. In other specific embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, and most preferably are 60 nucleotides in length.

The probes may comprise DNA or DNA "mimics" (e.g., derivatives and analogues) corresponding to a portion of an organism's genome. In another embodiment, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone. Exemplary DNA mimics include, e.g., phosphorothioates.

DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). Typically each probe on the microarray will be between 10 bases and 50,000 bases, usually between 300 bases and 1,000 bases in length. PCR methods are well known in the art, and are described, for example, in Innis et al., eds., PCR PROTOCOLS: A GUIDE TO METHODS AND APPLICATIONS, Academic Press Inc., San Diego, Calif. (1990). It will be apparent to one skilled in the art that controlled robotic systems are useful for isolating and amplifying nucleic acids.

An alternative, preferred means for generating the polynucleotide probes of the microarray is by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some embodiments, synthetic nucleic acids include non-natural bases, such as, but by no means limited to, inosine. As noted above, nucleic acid analogues may be used as binding sites for hybridization. An example of a suitable nucleic acid analogue is peptide nucleic acid (see, e.g., Egholm et al., Nature 363:566-568 (1993); U.S. Pat. No. 5,539,083).

Probes are preferably selected using an algorithm that takes into account binding energies, base composition, sequence complexity, cross-hybridization binding energies, and secondary structure. See Friend et al., International Patent Publication WO 01/05935, published Jan. 25, 2001; Hughes et al., Nat. Biotech. 19:342-7 (2001).

A skilled artisan will also appreciate that positive control probes, e.g., probes known to be complementary and hybridizable to sequences in the target polynucleotide molecules, and negative control probes, e.g., probes known to not be complementary and hybridizable to sequences in the target polynucleotide molecules, should be included on the array. In one embodiment, positive controls are synthesized along the perimeter of the array. In another embodiment, positive controls are synthesized in diagonal stripes across the array. In still another embodiment, the reverse complement for each probe is synthesized next to the position of the probe to serve as a negative control. In yet another embodiment, sequences from other species of organism are used as negative controls or as "spike-in" controls.

The probes are attached to a solid support or surface, which may be made, e.g., from glass, plastic (e.g., polypropylene, nylon), polyacrylamide, nitrocellulose, gel, or other porous or nonporous material. A preferred method for attaching the nucleic acids to a surface is by printing on glass plates, as is described generally by Schena et al, Science 270:467-470 (1995). This method is especially useful for preparing microarrays of cDNA (See also, DeRisi et al, Nature Genetics 14:457-460 (1996); Shalon et al., Genome Res. 6:639-645 (1996); and Schena et al., Proc. Natl. Acad. Sci. U.S.A. 93:10539-11286 (1995)).

A second preferred method for making microarrays is by making high-density oligonucleotide arrays. Techniques are known for producing arrays containing thousands of oligonucleotides complementary to defined sequences, at defined locations on a surface using photolithographic techniques for synthesis in situ (see, Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (Blanchard et al., Biosensors & Bioelectronics 11:687 -690). When these methods are used, oligonucleotides (e.g., 60-mers) of known sequence are synthesized directly on a surface such as a derivatized glass slide. Usually, the array produced is redundant, with several oligonucleotide molecules per RNA.

Other methods for making microarrays, e.g., by masking (Maskos and Southern, 1992, Nuc. Acids. Res. 20: 1679-1684), may also be used. In principle, and as noted supra, any type of array, for example, dot blots on a nylon hybridization membrane (see Sambrook et al., MOLECULAR CLONING--A LABORATORY MANUAL (2ND ED.), Vols. 1-3, Cold Spring Harbor Laboratory, Cold Spring Harbor, N.Y. (1989)) could be used. However, as will be recognized by those skilled in the art, very small arrays will frequently be preferred because hybridization volumes will be smaller.

In one embodiment, the arrays are prepared by synthesizing polynucleotide probes on a support. In such an embodiment, polynucleotide probes are attached to the support covalently at either the 3' or the 5' end of the polynucleotide.

In a particularly preferred embodiment, micro arrays are manufactured by means of an ink jet printing device for oligonucleotide synthesis, e.g., using the methods and systems described by Blanchard in U.S. Pat. No. 6,028,189; Blanchard et al., 1996, Biosensors and Bioelectronics 11:687-690; Blanchard, 1998, in Synthetic DNA Arrays in Genetic Engineering, Vol. 20, 1. K. Setlow, Ed., Plenum Press, New York at pages 111-123. Specifically, the oligonucleotide probes in such micro arrays are preferably synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells, which define the locations of the array elements (i.e., the different probes). Microarrays manufactured by this ink-jet method are typically of high density, preferably having a density of at least about 2,500 different probes per 1 cm.sup.2. The polynucleotide probes are attached to the support covalently at either the 3' or the 5' end of the polynucleotide.

The polynucleotide molecules which may be analyzed by the present invention are DNA, and optionally RNA or protein. The target polynucleotides are detectably labeled at one or more nucleotides. Any method known in the art may be used to detectably label the target polynucleotides. Preferably, this labeling incorporates the label uniformly along the length of the DNA or RNA, and more preferably, the labeling is carried out at a high degree of efficiency.

Preferably, the detectable label is a luminescent label. For example, fluorescent labels, bioluminescent labels, chemiluminescent labels, and colorimetric labels may be used in the present invention. A highly preferred label is a fluorescent label, such as a fluorescein, a phosphor, a rhodamine, or a polymethine dye derivative. Examples of commercially available fluorescent labels include, for example, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.). In another embodiment, the detectable label is a radiolabeled nucleotide.

In a further preferred embodiment, target polynucleotide molecules from a patient sample are labeled differentially from target polynucleotide molecules of a reference sample. The reference can comprise target polynucleotide molecules from normal tissue samples.

Nucleic acid hybridization and wash conditions are chosen so that the target polynucleotide molecules specifically bind or specifically hybridize to the complementary polynucleotide sequences of the array, preferably to a specific array site, wherein its complementary DNA is located.

Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self complementary sequences.

Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook et al., MOLECULAR CLONING--A LABORATORY MANUAL (2ND ED.), Vols. 1-3, Cold Spring Harbor Laboratory, Cold Spring Harbor, N.Y. (1989), and in Ausubel et al., CURRENT PROTOCOLS IN MOLECULAR BIOLOGY, vol. 2, Current Protocols Publishing, New York (1994). Typical hybridization conditions for the cDNA microarrays of Schena et al. are hybridization in 5 x SSC plus 0.2% SDS at 65°C for four hours, followed by washes at 25° C in low stringency wash buffer (1 x SSC plus 0.2% SDS), followed by 10 minutes at 25°C in higher stringency wash buffer (0.1 x SSC plus 0.2% SDS) (Schena et al., Proc. Nati. Acad. Sci. U.S.A. 93:10614 (1993)). Useful hybridization conditions are also provided in, e.g., Tijessen, 1993, HYBRIDIZATION WITH NUCLEIC ACID PROBES, Elsevier Science Publishers B.V.; and Kricka, 1992, NONISOTOPIC DNA PROBE TECHNIQUES, Academic Press, San Diego, Calif.

Particularly preferred hybridization conditions include hybridization at a temperature at or near the mean melting temperature of the probes (e.g., within 51°C., more preferably within 21°C.) in 1 M NaCl, 50 mM MES buffer (pH 6.5), 0.5% sodium sarcosine and 30% formamide.

When fluorescently labeled genes or gene products are used, the fluorescence emissions at each site of a microarray may be, preferably, detected by scanning confocal laser microscopy. In one embodiment, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously (see Shalon et al., 1996, "A DNA micro array system for analyzing complex DNA samples using two-color fluorescent probe hybridization," Genome Research 6:639-645.

In a preferred embodiment, the arrays are scanned with a laser fluorescent scanner with a computer controlled X-Y stage and a microscope objective. Sequential excitation of the two fluorophores is achieved with a multi-line, mixed gas laser 'and the emitted light is split by wavelength and detected with two photomultiplier tubes. Fluorescence laser scanning devices are described in Schena et al., Genome Res. 6:639-645 (1996), and in other references cited herein. Alternatively, the fiber-optic bundle described by Ferguson et al., Nature Biotech. 14:1681-1684 (1996), may be used to monitor mRNA abundance levels at a large number of sites simultaneously.

### EXAMPLES

### Example 1 - Identification of oocyte proteins

Oocytes are collected from females, for example mice, by superovulation, and zona pellucidae are removed by treatment with acid Tyrode solution. Oocyte plasma membrane (oolemma) proteins exposed on the surface can be distinguished at this point by biotin labeling. The treated oocytes are washed in 0.01 M PBS and treated with lysis buffer (7 M urea, 2 M thiourea, 4% (w/v) 3-[(3-cholamidopropyl)dimethylammonio]-I-propanesulfonate (CHAPS), 65 mM dithiothreitol (DTT), and 1% (v/v) protease inhibitor at -80°C). Oocyte proteins are resolved by one-dimensional or two-dimensional SDS-PAGE. The gels are stained, visualized, and sliced. Proteins in the gel pieces are digested (12.5 ng/µl trypsin in 50 mM ammonium bicarbonate overnight at 37°C), and the peptides are extracted and microsequenced.

### Example 2 - Sample Population for Identification of Infertilitv-Related Polymorphisms

Genomic DNA is collected from 30 female subjects (15 who have failed multiple rounds of IVF versus 15 who were successful). In particular, all of the subjects are under age 35. Members of the control group succeeded in conceiving through IVF. Members of the test group have a clinical diagnosis of idiopathic infertility, and have failed three of more rounds of IVF with no prior pregnancy. The women are able to produce eggs for IVF and have a reproductively normal male partner. To focus on infertility resulting from oocyte defects (and eliminate factors such as implantation defects) women who have subsequently conceived by egg donation are favored.

### Example 3 - Sample Population for Identification of Infertility-Related Polymorphisms

In a follow-up study of a larger cohort, genomic DNA is collected from 300 female subjects (divided into groups having profiles similar to the groups described above). The DNA sequence polymorphisms to be investigated are selected based on the results of small initial studies.

### Example 4 - Sample Population for Identification of Premature Ovarian Failure (POF) and Premature Maternal Aging Polymorphisms

Genomic DNA is collected from 30 female subjects who are experiencing symptoms of premature decline in egg quality and reserve including abnormal menstrual cycles or amenorrhea. In particular, all of the subjects are between the ages of 15-40 and have follicle stimulating hormone (FSH) levels of over 20 international units (IU) and a basal antral follicle count of under 5. Members of the control group succeeded in conceiving through IVF. Members of the test group have no previous history of toxic exposure to known fertility damaging treatments such as chemotherapy. Members of this group may also have one or more female family member who experienced menopause before the age of 40.

### Example 5 - Sample Procurement and Preparation

Blood is drawn from patients at fertility clinics for standard procedures such as gauging hormone levels and many clinics bank this material after consent for future research projects. Although DNA is easily obtained from blood, wider population sampling is accomplished using home-based, noninvasive methods of DNA collection such as saliva using an Oragene DNA self collection kit (DNA Genotek).

Blood samples - Three-milliliter whole blood samples are venously collected and treated with sodium citrate anticoagulant and stored at 4 °C until DNA extraction.

Whole Saliva - Whole saliva is collected using the Oragene DNA selfcollection kit following the manufacturer's instructions. Participants are asked to rub their tongues around the inside of their mouths for about 15 sec and then deposit approximately 2 ml saliva into the collection cup. The collection cup is designed so that the solution from the vial.'s lower compartment is released and mixes with the saliva when the cap is securely fastened. This starts the initial phase of DNA isolation, and stabilizes the saliva sample for long-term storage at room temperature or in low temperature freezers. Whole saliva samples are stored and shipped, if necessary, at room temperature. Whole saliva has the potential advantage over other non-invasive DNA sampling methods, such as buccal and oral rinse, of providing large numbers of nucleated cells (eg., epithelial cells, leukocytes) per sample.

Blood clots - Clotted blood that is usually discarded after extraction through serum separation, for other laboratory tests such as for monitoring reproductive hormone levels is collected and stored at -80 °C until extraction.

Sample Preparation - Genomic DNA is prepared from patient blood or saliva for downstream sequencing applications with commercially available kits (e.g., Invitrogen.'s ChargeSwitch® gDNA Blood Kit or DNA Genotek kits, respectively). Genomic DNA from clotted is prepared by standard methods involving proteinase K digestion, salt/chloroform extraction and 90% ethanol precipitation of DNA. (see N Kanai et al., 1994, " Rapid and simple method for preparation of genomic DNA from easily obtainable clotted blood," J Clin Pathol 47:1043-1044).

### Example 6 - Manufacturing of a Customized Oligonucleotide Library

A customized oligonucleotide library is used to enrich samples for DNAs encoding proteins of interest. Agilent 's eArray (a web-based design tool) is used to created a customized target enrichment system tailored to infertility related genes. A customized library of 55,000 oligos (120mers) (which covers a 3.3mb chromosomal region) is designed to target genes of Table 1. The custom RNA oligonucleotides, or baits, are biotinylated for easy capture onto streptavidin-labeled magnetic beads and used in Agilent.'s SureSelect Target Enrichment System.

The target enrichment procedure uses an extremely efficient hybrid selection technique, and significantly improves the cost- and process efficiency of the sequencing workflow. Target sequence enrichment ensures that only the genomic areas of interest can be sequenced, creating process efficiencies that reduce costs and permit more samples to be analyzed per study. The SureSelect Target Enrichment System workflow is solution-based and is performed in microcentrifuge tubes or microtiter plates.

### Example 7 - Capture of Genomic DNA

Genomic DNA is sheared and assembled into a library format specific to the sequencing instrument utilized downstream. Size selection is performed on the sheared DNA and confirmed by electrophoresis or other size detection method. The size-selected DNA is incubated with biotinylated RNA oligonucleotides "baits" for 24 hours. The RNA/DNA hybrids are immobilized to streptavidin-labeled magnetic beads, which are captured magnetically. The RNA baits are then digested, leaving only the target selected DNA of interest, which is then amplified and sequenced.

### Example 8 - Sequencing of Target Selected DNA

Target-selected DNA is sequenced by a paired end (50bp) re-sequencing procedure using Illumina.'s Genome Analyzer. The combined DNS targeting and resequencing provides 45 fold redundancy which is greater than the accepted industry standard for SNP discovery.

### Example 9 - Correlation of Polymorphisms with Fertility

Polymorphisms among the sequences of target selected DNA from the pool of test subjects are identified, and may be classified according to where they occur in promoters, splice sites, or coding regions of a gene. Polymorphisms can also occur in regions that have no apparent function, such as introns and upstream or downstream non-coding regions. Although such polymorphisms may not be informative as to the functional defect of an allele, nevertheless, they are linked to the defect and useful for predicting infertility. The polymorphisms are analyzed statistically to determine their correlation with the fertility status of the test subjects. The statistical analysis indicates that certain polymorphisms identify gene defects that by themselves (homozygous or heterozygous) are sufficient to cause infertility. Other polymorphisms identify genetic variants that reduce, but do not eliminate fertility. Other polymorphisms identify genetic variants that have an apparent effect on fertility only in the presence of particular variants of other genes. Other polymorphisms identify genetic variants that have an apparent effect on fertility only in the presence of particular phenotypes. Other polymorphisms identify genetic variants that have an apparent effect on fertility only in the presence of particular environmental exposures. Still other polymorphisms identify genetic variants that have an apparent effect on fertility only in the presence of any combination of particular variants of other genes, presence of particular phenotypes, and particular environmental exposures.

### Example 10 - Correlation of Polymorphisms with Premature Ovarian Failure (POF)

Polymorphisms among the sequences of target selected DNA from the pool of test subjects are identified, and may be classified according to where they occur in promoters, splice sites, or coding regions of a gene. Polymorphisms can also occur in regions that have no apparent function, such as introns and upstream or downstream non-coding regions. Although such polymorphisms may not be informative as to the functional defect of an allele, nevertheless, they are linked to the defect and useful for predicting likelihood of premature ovarian failure (POF). The polymorphisms are analyzed statistically to determine their correlation with the POF status of the test subjects. The statistical analysis indicates that certain polymorphisms identify gene defects that by themselves (homozygous or heterozygous) are sufficient to cause POF. Other polymorphisms identify genetic variants that increase the likelihood, but do not cause POF. Other polymorphisms identify genetic variants that have an apparent effect on POF only in the presence of particular variants of other genes. Other polymorphisms identify genetic variants that have an apparent effect on POF only in the presence of particular phenotypes. Other polymorphisms identify genetic variants that have an apparent effect on POF only in the presence of particular environmental exposures. Still other polymorphisms identify genetic variants that have an apparent effect on POF only in the presence of any combination of particular variants of other genes, presence of particular phenotypes, and particular environmental exposures.

### Example 11 - Correlation of Polymorphisms with Premature Maternal Aging

Polymorphisms among the sequences of target selected DNA from the pool of test subjects are identified, and may be classified according to where they occur in promoters, splice sites, or coding regions of a gene. Polymorphisms can also occur in regions that have no apparent function, such as introns and upstream or downstream non-coding regions. Although such polymorphisms may not be informative as to the functional defect of an allele, nevertheless, they are linked to the defect and useful for predicting likelihood of premature decline in ovarian reserve and egg quality (i.e. maternal aging). The polymorphisms are analyzed statistically to determine their correlation with the maternal aging status of the test subjects. The statistical analysis indicates that certain polymorphisms identify gene defects that by themselves (homozygous or heterozygous) are sufficient to cause premature maternal aging. Other polymorphisms identify genetic variants that increase the likelihood, but do not cause premature maternal aging. Other polymorphisms identify genetic variants that have an apparent effect on premature maternal aging only in the presence of particular variants of other genes. Other polymorphisms identify genetic variants that have an apparent effect on premature maternal aging only in the presence of particular phenotypes. Other polymorphisms identify genetic variants that have an apparent effect on premature maternal aging only in the presence of particular environmental exposures. Still other polymorphisms identify genetic variants that have an apparent effect on premature maternal aging only in the presence of any combination of particular variants of other genes, presence of particular phenotypes, and particular environmental exposures.

### Example 12 - Diagnostics and Counseling

A library of nucleic acids in an array format is provided for infertility diagnosis. The library consists of selected nucleic acids for enrichment of genetic targets wherein polymorphisms in the targets are correlated with variations in fertility. A patient nucleic acid sample (appropriately cleaved and size selected) is applied to the array, and patient nucleic acids that are not immobilized are washed away. The immobilized nucleic acids of interest are then eluted and sequenced to detect polymorphisms. According to the polymorphisms detected, the fertility status of the patient is evaluated and/or quantified. The patient is accordingly advised as to the suitability and likelihood of success of a fertility treatment or suitability or necessity of a particular *in vitro* fertilization procedure.

### Example 13 - Diagnostics and Counseling

A complete DNA sequence of any number of or all of the genes in Table 1 is determined using a targeted resequencing protocol. According to the polymorphisms detected and the phenotypic traits and environmental exposures reported, the fertility status of the patient is evaluated and/or quantified. The patient is accordingly advised as to the suitability and likelihood of success of a fertility treatment or suitability or necessity of a particular *in vitro* fertilization procedure.

### Example 14 - Diagnostics and Counseling

A library of nucleic acids in an array format is provided for infertility diagnosis. The library consists of selected nucleic acids for enrichment of genetic targets wherein polymorphisms in the targets are correlated with variations in fertility. A patient nucleic acid sample (appropriately cleaved and size selected) is applied to the array, and patient nucleic acids that are not immobilized are washed away. The immobilized nucleic acids of interest are then eluted and sequenced to detect polymorphisms. According to the polymorphisms detected and the phenotypic traits and environmental exposures reported, the POF status of the patient or likelihood of future POF occurrence is evaluated and/or quantified. The patient is accordingly advised as to whether preventative egg or ovary preservation is indicated.

### Example 15 - Diagnostics and Counseling

A complete DNA sequence of any number of or all of the genes in Table 1 is determined using a targeted resequencing protocol. According to the polymorphisms detected and the phenotype and environmental exposures reported, the fertility status of the patient is evaluated and/or quantified. According to the polymorphisms detected and the phenotypic traits and environmental exposures reported, the POF status of the patient or likelihood of future POF occurrence is evaluated and/or quantified. The patient is accordingly advised as to whether preventative egg or ovary preservation is indicated.

### Example 16 - Diagnostics and Counseling

A library of nucleic acids in an array format is provided for infertility diagnosis. The library consists of selected nucleic acids for enrichment of genetic targets wherein polymorphisms in the targets are correlated with variations in fertility. A patient nucleic acid sample (appropriately cleaved and size selected) is applied to the array, and patient nucleic acids that are not immobilized are washed away. The immobilized nucleic acids of interest are then eluted and sequenced to detect polymorphisms. According to the polymorphisms detected and the phenotypic traits and environmental exposures reported, the maternal aging status of the patient or likelihood of future premature maternal aging occurrence is evaluated and/or quantified. The patient is accordingly advised as to whether preventative egg or ovary preservation, minimization of certain environmental exposures such as alcohol intake or smoking, or mitigation of certain phenotypes such as having children at a younger age is indicated.

### Example 17 - Diagnostics and Counseling

A complete DNA sequence of any number of or all of the genes in Table 1 is determined using a targeted resequencing protocol. According to the polymorphisms detected and the phenotypic traits and environmental exposures reported, the fertility status of the patient is evaluated and/or quantified. According to the polymorphisms detected and the phenotype and environmental exposures reported, the maternal aging status of the patient or likelihood of future premature maternal aging occurrence is evaluated and/or quantified. The patient is accordingly advised as to whether preventative egg or ovary preservation, minimization of certain environmental exposures such as alcohol intake or smoking, or mitigation of certain phenotypes such as having children at a younger age is indicated.

## Claims

1. A method for assessing infertility and/or egg quality in a post-pubescent mammal sample excluding a human embryonic sample, the method comprising:
a) conducting a sequencing assay that detects variation in at least six infertility-associated maternal effect genes from Table 1:
| **Table 1 - Human Fertility-Related Genes (OMIM ref # / Alternative Designations** | | |
|---|---|---|
| PADI6 | Pla2g4c | PROKI |
| (610363) | (603602) | (606233) |
| NPM2 | TACC3 | PROKRI |
| (608073) | (605303) | (607122) |
| NLRP5 | C60RF221 | PROKR2 |
| (609658 / MATER) | (611687 / FILIA) | (607123) |
| SMARCA4 | TLE6 | DPPA5 |
| (607123/ Brgl) | (612399) | (611111) |
| OOEP | AURKB | ACTL6A |
| (611689/ FlopedlMOEP19) | (604970) | (604958) |
| HSFI | AURKA | CARMI |
| (140580) | (603072) | (603934) |
| ZARI | DDX20 | SPIN |
| (607520) | (606168) | (609936) |
| DPPA3 | FIGLA | OAS1 |
| (608408/ Stella) | (608697) | (164350) |
| DNMT1 | ZP1 | VIL2 |
| (126375) | (195000) | (123900) |
| CTCF | ZP2 | NLRP14 |
| (604167) | (182888) | (609665) |
| PMS2 | ZP3 | CD55 |
| (600259) | (182889) | (125240) |
| BNC1 | IL-10 | IFITM1 |
| (601930) | (124092) | (604456 / Fragi1is) |
| BUB1 | KDM1B | GDF3 |
| (602452) | (613081) | (606522) |
| GDF1 | REX 1 | CDX2 |
| (602880) | (609614) | (600297) |
| BMP4 | KLF4 | EZH2 |
| (112262) | (602253) | (601573) |
| SUZ12 | SOX17 | MYC |
| (606245) | (610928) | (190080) |
| P53 | DDX43 | C60RF221 |
| (191170) | (606286) | (611687) |
| BRCA1 | EEF1A1 | BRCA2 |
| (113705) | (130590) | (600185) |
| FMR1 | DIAPH2 | HS6ST1 |
| (309550) | (300108) | (604846) |
| GPC3 | FOXL2 | GALT |
| (300037) | (605597) | (606999) |
| FMR2 | BMP15 | FSHR |
| (300806) | (300247) | (136435) |
| LHCGR | FSHB | UBE3A |
| (152790) | (136530) | (601623) |
| LHB | INHA | AIRE |
| (152780) | (147380) | (607358) |
| EIF2B2 | EIF2B4 | EIF2B5 |
| (606454) | (606687) | (603945) |
| NOG | BMP7 | POLG |
| (602991) | (112267) | (174763) |
| USP9X | XPNPEP2 | ZFX |
| (300072) | (300145) | (314980) |
| CENPI | MADILI | CENPF |
| (300065) | (602686) | (600236) |
| MAD1L2 | XIST | CDX4 |
| (601467) | (314670) | (300025) |
| WT1 | ATM | ATR |
| (607102) | (607585) | (601215) |
| GDF9 | NOBOX | RFPL4A |
| (601918) | (610934) | (612601) |
| GREM2 | FOXE1 | BAX |
| (608832) | (602617) | (600040) |
| AHR | | |
| (600253) | | |
;
b) determining at least one infertility-associated phenotypic trait of the post-pubescent mammal or environmental exposure at least one of which is selected from Table 2:
| **Table 2 - Phenotypic and environmental variables impacting fertility success** |
|---|
| Cholesterol levels on different days of the menstrual cycle |
| Age of first menses for patient, sisters, mother, grandmothers |
| Age of menopause for sisters, mother, grandmothers |
| Number of previous pregnancies (biochemical/ectopic/clinical/fetal heart beat detected), age at the time, and outcome for patient, sisters, mother, grandmothers |
| Polycystic ovarian syndrome |
| History of hydrosalpinx or tubal occlusion |
| History of endometriosis or painful periods |
| Cancer history/type of cancer/treatment/outcome for patient, sisters, mother, grandmothers |
| Age that sexual activity began, current level of sexual activity |
| Smoking history for patient, sisters, mother, grandmothers |
| Travel schedule/number of flying hours a year/time difference changes of more than 3 hours (jetlag) |
| Nature of period (length of menses, length of cycle) |
| Biological age (number of years since first menses) |
| Birth control use |
| Drug use (illegal or legal) |
| Body mass index (current, lowest ever, highest ever) |
| History of polyps |
| History of hormonal imbalance |
| History of amenorrhoea |
| History of eating disorders |
| Alcohol consumption by patient, sisters, mother, grandmothers |
| Details of mother's pregnancy with patient: any drugs taken, smoking, alcohol, stress levels, exposure to plastics (i.e. Tupperware), composition of diet (see below) |
| Sleep patterns: number of hours a night, continuous/overall |
| Diet: meat, organic produce, vegetables, vitamin or other supplement consumption, dairy (full fat or reduced fat), coffee/tea consumption, folic acid, sugar (complex, artificial, simple), processed food versus home cooked. |
| Exposure to plastics: microwave in plastic, cook with plastic, store food in plastic, plastic water or coffee mugs. |
| Water consumption: amount per day, format: straight from the tap, bottled water (plastic or bottle), filtered (type Britta/Pur) |
| Residence history starting with mother's pregnancy: location/duration |
| Environmental exposure to potential toxins for different regions (extracted from government monitoring databases) |
| Health metrics: autoimmune disease, chronic illness/condition |
| Pelvic surgery history |
| Life time number of pelvic X-rays |
| History of sexually transmitted infections: type/treatment/outcome |
| Reproductive hormone levels: follicle stimulating hormone, anti-Mullerian hormone, estrogen, progesterone |
| Stress |
| Thickness and type of endometrium throughout the menstrual cycle. |
| Age |
| Height |
| Fertility treatment history and details: history of hormone stimulation, brand of drugs used, basal antral follicle count, follicle count after stimulation with different protocols, number/quality/stage of retrieved oocytes/ development profile of embryos resulting in vitro insemination (natural or ICSI), details oflVF procedure (which clinic, doctor/embryologist at clinic, assisted hatching, fresh or thawed oocytes/embryos, embryo transfer (blood on the catheter/squirt detection and direction on ultrasound) |
| Morning sickness during pregnancy |
| Breast size before/during/after pregnancy |
| History of ovarian cysts |
| Twin or sibling from multiple birth (mono-zygotic or di-zygotic) |
| Male factor infertility for reproductive partner: Semen analysis (count, motility,morphology), Vasectomy, male cancer, smoking, alcohol, diet, STIs |
; and
c) conducting an association study based on results from steps (a) and (b) using weighted predictor variables to assess infertility and/or egg quality in the post-pubescent mammal.

2. A method as claimed in claim 1, further comprising conducting an assay on an abnormal gene product.

3. A method as claimed inclaim 1, wherein the variation is selected from the group consisting of: a single nucleotide polymorphism; a deletion; an insertion; a rearrangement; a copy number variation; and a combination thereof.

4. A method as claimed in claim 2, wherein the gene product is a product of a maternal effect gene.

5. A method as claimed in claim 4, wherein the maternal effect gene is a gene from Table 1.

6. A method as claimed inclaim 2, wherein the gene product is RNA or protein.

7. A method as claimed inclaim 4, wherein the assay comprises determining an amount of the gene product and comparing the determined amount to a reference.

## Patentansprüche

1. Verfahren zur Beurteilung der Unfruchtbarkeit und/oder der Eiqualität in einer Probe eines post-pubertierenden Säugetiers, exklusive einer embryonalen Probe, wobei das Verfahren folgendes umfasst:
a) Ausführen eines Sequenzierungsassays, der Variationen in mindestens sechs mit Unfruchtbarkeit assoziierten maternalen Effektgenen aus Tabelle 1:
| **Tabelle 1-Mit menschlicher Fruchtbarkeit assoziierte Gene (OMIM Ref#/ Alternative Bezeichnungen** | | |
|---|---|---|
| PADI6 | PIa2g4c | PROKI |
| (610363) | (603602) | (606233) |
| NPM2 | TACC3 | PROKRI |
| (608073) | (605303) | (607122) |
| NLRP5 | C60RF221 | PROKR2 |
| (609658 / MATER) | (611687 / FILIA) | (607123) |
| SMARCA4 | TLE6 | DPPAS |
| (607123 / Brgl) | (612399) | (611111) |
| OOEP | AURKB | ACTL6A |
| (611689 / FlopedIMOEP19) | (604970) | (604958) |
| HSFI | AURKA | CARMI |
| (140580) | (603072) | (603934) |
| ZARI | DDX20 | SPIN |
| (607520) | (606168) | (609936) |
| DPPA3 | FIGLA | OAS1 |
| (608408 / Stella) | (608697) | (164350) |
| DNMT1 | ZP1 | VIL2 |
| (126375) | (195000) | (123900) |
| CTCF | ZP2 | NLRP14 |
| (604167) | (182888) | (609665) |
| PMS2 | ZP3 | CD55 |
| (600259) | (182889) | (125240) |
| BNC1 | IL-10 | IFITM1 |
| (601930) | (124092) | (604456 / Fragi1 is) |
| BUB1 | KDM1B | GDF3 |
| (602452) | (613081) | (606522) |
| GDF1 | REX 1 | CDX2 |
| (602880) | (609614) | (600297) |
| BMP4 | KLF4 | EZH2 |
| (112262) | (602253) | (601573) |
| SUZ12 | SOX17 | MYC |
| (606245) | (610928) | (190080) |
| P53 | DDX43 | C60RF221 |
| (191170) | (606286) | (611687) |
| BRCA1 | EEF1A1 | BRCA2 |
| (113705) | (130590) | (600185) |
| FMR1 | DIAPH2 | HS6ST1 |
| (309550) | (300108) | (604846) |
| GPC3 | FOXL2 | GALT |
| (300037) | (605597) | (606999) |
| FMR2 | BMP15 | FSHR |
| (300806) | (300247) | (136435) |
| LHCGR | FSHB | UBE3A |
| (152790) | (136530) | (601623) |
| LHB | INHA | AIRE |
| (152780) | (147380) | (601623) |
| EIF2B2 | EIF2B4 | EIF2B5 |
| (606454) | (606687) | (603945) |
| NOG | BMP7 | POLG |
| (602991) | (112267) | (174763) |
| USP9X | XPNPEP2 | ZFX |
| (300072) | (300145) | (314980) |
| CENPI | MADILI | CENPF |
| (300065) | (602686) | (600236) |
| MAD1L2 | XIST | CDX4 |
| (601467) | (314670) | (300025) |
| WT1 | ATM | ATR |
| (607102) | (607585) | (601215) |
| GDF9 | NOBOX | RFPL4A |
| (601918) | (610934) | (612601) |
| GREM2 | FOXE1 | BAX |
| (608832) | (602617) | (600040) |
| AHR | | |
| (600253) | | |
;
b) Bestimmen mindestens einer mit Unfruchtbarkeit assoziierten phänotypischen Eigenschaft des postpubertierenden Säugetiers oder der Umweltbelastung, wovon mindestens eines aus Tabelle 2 ausgewählt ist:
| **Tabelle 2** - **Phänotypische und Umweltvariablen, welche den Fruchtbarkeitserfolg beeinflussen** |
|---|
| Cholesterinwerte an unterschiedlichen Tagen des Menstruationszyklus |
| Alter bei erster Menstruation der Patientin, deren Schwestern, Mutter, Großmüttern |
| Alter bei Menopause der Schwestern, Mutter, Großmütter |
| Anzahl bisheriger Schwangerschaften (biochemisch/ektopisch/klinisch/nachgewiesener Herzschlag eines Fötus), Alter zu dem Zeitpunkt und Ergebnis bei der Patientin, deren Schwestern, Mutter, Großmüttern |
| Syndrom der Polyzistischen Ovarien |
| Historie von Hydrosalpinx oder Eileiterverschluss |
| Historie von Endometriose oder schmerzhaften Perioden |
| Krebshistorie/Krebsart/Behandlung/Ergebnis bei der Patientin, deren Schwestern, Mutter, Großmüttern |
| Alter zu Beginn sexueller Aktivität, aktueller Stand sexueller Aktivität |
| Rauchhistorie bei der Patientin, deren Schwestern, Mutter, Großmüttern |
| Reiseplan/Anzahl der Flugstunden pro Jahr/Zeitverschiebungen von über 3 Stunden (Jetlag) |
| Art der Periode (Länge der Menses, Länge des Zyklus) |
| Biologisches Alter (Anzahl der Jahre nach der ersten Menstruation) |
| Eingesetzte Verhütungsmittel |
| Arzneimittel-/Drogenkonsum (illegal oder legal) |
| Body-Mass-Index (aktuell, niedrigster bisheriger Wert, höchster bisheriger Wert) |
| Polypen historie |
| Historie des hormonellen Ungleichgewichts |
| Historie der Amenorrhö |
| Historie von Essstörungen |
| Alkoholkonsum der Patientin, deren Schwestern, Mutter, Großmüttern |
| Einzelheiten der Schwangerschaft der Mutter bei Schwangerschaft mit der Patientin: etwaige eingenommene Drogen/Arzneimittel, Rauchverhalten, Alkohol, Stresslevel, Kontakt mit Kunststoffen (z.B. Tupperware), Ernährungszusammensetzung (siehe unten) |
| Schlafverhalten: Anzahl der Stunden pro Nacht, unterbrechungsfrei/insgesamt |
| Ernährung: Fleisch, organische Nahrungsmittel, Gemüse, Konsum von Vitaminen oder Nahrungsergänzungsmitteln, Milchprodukte (voller Fettgehalt oder fettreduziert), Konsum von Kaffee/Tee, Folsäure, Zucker (Komplex, Süßstoffe, einfach), industriell verarbeitete Lebensmittel im Vergleich zu selbstgekochtem Essen |
| Kontakt mit Kunststoffen: Mikrowellenkost in Kunststoff, Kochen mit Kunststoffen, Aufbewahrung von Nahrungsmitteln in Kunststoffen, Wasser in Kunststoffverpackungen oder Kaffeetassen aus Kunststoff |
| Wasserkonsum: Menge pro Tag, Form: direkt aus dem Wasserhahn, Wasser aus Flaschen (Kunststoff oder Glas), gefiltert (Britta-Filter/Pur) |
| Wohnorthistorie beginnend mit der Schwangerschaft der Mutter: Ort/Dauer |
| Umweltbelastung durch potentielle Toxine für verschiedene Regionen (offiziellen staatlichen Datenbanken entnommen) |
| Gesundheitsdaten: Autoimmunerkrankungen, chronische Erkrankungen/Zustände |
| Historie chirurgischer Eingriffe am Becken |
| Anzahl bisheriger Röntgenaufnahmen der Beckenregion |
| Historie durch sexuelle Kontakte übertragener Infektionen: Typ/Behandlung/Ergebnis |
| Werte der Sexualhormone: follikelstimulierendes Hormon, Anti-Müller-Hormon, Östrogen, Progesteron |
| Stress |
| Dicke und Art des Endometriums während Menstruationszyklus |
| Alter |
| Körpergröße |
| Verlauf und Einzelheiten der Fruchtbarkeitsbehandlung: Historie der Hormonstimulation, Hersteller der konsumierten Drogen/Arzneimittel, Tertiärfollikelzahl, Follikelzahl nach Stimulation mit verschiedenen Protokollen, Anzahl/Qualität/Stadium gewonnener Oozyten/Entwicklungsprofil von Embryos aus in-vitro-Befruchtung (natürlich oder ICSI), Einzelheiten von ofIVF-Prozedur (welche Klinik, welcher Arzt/Embryologe in Klinik, Assisted Hatching, frische oder aufgetaute Oozyten/Embryos, Embryotransfer (Blut an Katheter/Squirt-Nachweis und Richtung des Ultraschalls) |
| Morgendliche Übelkeit während Schwangerschaft |
| Brustgröße vor/während/nach Schwangerschaft |
| Zwilling oder Geschwister aus Mehrlingsgeburt (monozygotisch oder dizygotisch) |
| Männlicher Faktor der Unfruchtbarkeit für Fortpflanzungspartner: Spermiogramm (Anzahl, Motilität, Morphologie), Vasektomie, Krebs beim Mann, Rauchverhalten, Alkoholkonsum, Ernährung, STIs |
; und
c) Durchführen einer Analyse auf der Basis der Ergebnisse der Schritte (a) und (b) unter Verwendung gewichteter Einflussvariablen zur Einschätzung der Unfruchtbarkeit und/oder Eiqualität in dem post-pubertierenden Säugetier.

2. Verfahren nach Anspruch 1, welches ferner das Durchführen eines Assays an einem anomalen Genprodukt umfasst.

3. Verfahren nach Anspruch 1, wobei die Variation ausgewählt ist aus der Gruppe bestehend aus: einem Einzelnukleotid-Polymorphismus; einer Deletion; einer Insertion; einer Neuanordnung; einer Kopienzahlvariation und einer Kombination davon.

4. Verfahren nach Anspruch 2, wobei das Genprodukt ein Produkt eines maternalen Effektgens ist.

5. Verfahren nach Anspruch 4, wobei das maternale Effektgen ein Gen aus Tabelle 1 ist.

6. Verfahren nach Anspruch 2, wobei das Genprodukt RNA oder Protein ist.

7. Verfahren nach Anspruch 4, wobei der Assay das Bestimmen einer Menge des Genprodukts umfasst sowie das Vergleichen der bestimmten Menge mit einem Referenzwert.

## Revendications

1. Procédé d'évaluation de l'infertilité et/ou de la qualité des ovules dans un échantillon de mammifère post-pubère excluant un échantillon embryonnaire humain, le procédé comprenant les étapes consistant à :
a) effectuer un test de séquençage qui détecte la variation d'au moins six gènes d'effet maternel associés à l'infertilité dans le tableau 1 :
| **Tableau 1 - Gènes liés à la fertilité humaine (OMIM réf. # / Autres désignations)** | | |
|---|---|---|
| PADI6 | Pla2g4c | PROKI |
| (610363) | (603602) | (606233) |
| NPM2 | TACC3 | PROKRI |
| (608073) | (605303) | (607122) |
| NLRP5 | C60RF221 | PROKR2 |
| (609658/MATER) | (611687 / FILIA) | (607123) |
| SMARCA4 | TLE6 | DPPA5 |
| (607123/ Brgl) | (612399) | (611111) |
| OOEP | AURKB | ACTL6A |
| (611689/ FlopedlMOEP19) | (604970) | (604958) |
| HSFI | AURKA | CARMI |
| (140580) | (603072) | (603934) |
| ZARI | DDX20 | SPIN |
| (607520) | (606168) | (609936) |
| DPPA3 | FIGLA | OAS1 |
| (608408/ Stella) | (608697) | (164350) |
| DNMT1 | ZP1 | VIL2 |
| (126375) | (195000) | (123900) |
| CTCF | ZP2 | NLRP14 |
| (604167) | (182888) | (609665) |
| PMS2 | ZP3 | CD55 |
| (600259) | (182889) | (125240) |
| BNC1 | IL-10 | IFITM1 |
| (601930) | (124092) | (604456 / Fragilis) |
| BUB1 | KDM1B | GDF3 |
| (602452) | (613081) | (606522) |
| GDF 1 | REX1 | CDX2 |
| (602880) | (609614) | (600297) |
| BMP4 | KLF4 | EZH2 |
| (112262) | (602253) | (601573) |
| SUZ12 | SOX17 | MYC |
| (606245) | (610928) | (190080) |
| P53 | DDX43 | C60RF221 |
| (191170) | (606286) | (611687) |
| BRCA1 | EEF1A1 | BRCA2 |
| (113705) | (130590) | (600185) |
| FMR1 | DIAPH2 | HS6ST1 |
| (309550) | (300108) | (604846) |
| GPC3 | FOXL2 | GALT |
| (300037) | (605597) | (606999) |
| FMR2 | BMP15 | FSHR |
| (300806) | (300247) | (136435) |
| LHCGR | FSHB | UBE3A |
| (152790) | (136530) | (601623) |
| LHB | INHA | AIRE |
| (152780) | (147380) | (607358) |
| EIF2B2 | EIF2B4 | EIF2B5 |
| (606454) | (606687) | (603945) |
| NOG | BMP7 | POLG |
| (602991) | (112267) | (174763) |
| USP9X | XPNPEP2 | ZFX |
| (300072) | (300145) | (314980) |
| CENPI | MADILI | CENPF |
| (300065) | (602686) | (600236) |
| MAD1L2 | XIST | CDX4 |
| (601467) | (314670) | (300025) |
| WT1 | ATM | ATR |
| (607102) | (607585) | (601215) |
| GDF9 | NOBOX | RFPL4A |
| (601918) | (610934) | (612601) |
| GREM2 | FOXE1 | BAX |
| (608832) | (602617) | (600040) |
| AHR | | |
| (600253) | | |
;
b) déterminer au moins un caractère phénotypique associé à l'infertilité du mammifère post-pubère ou une exposition environnementale, dont au moins un est choisi dans le Tableau 2 :
| **Tableau 2 - Variables phénotypiques et environnementales influant sur le succès de la fécondité** |
|---|
| Taux de cholestérol à différents jours du cycle menstruel |
| Âge des premières menstrues pour la patiente, les sœurs, la mère, les grands-mères |
| Âge de la ménopause pour les sœurs, la mère, les grands-mères |
| Nombre de grossesses antérieures (biochimique/extrasystole/clinique/battement de cœur fœtal détecté), âge à l'époque et résultats pour la patiente, les sœurs, la mère, les grands-mères |
| Syndrome des ovaires polykystiques |
| Antécédents d'hydrosalpinx ou d'occlusion des trompes de Fallope |
| Antécédents d'endométriose ou de règles douloureuses |
| Antécédents de cancer/type de cancer/traitement/résultats pour la patiente, les sœurs, la mère, les grands-mères |
| Âge auquel l'activité sexuelle a commencé, niveau actuel de l'activité sexuelle |
| Antécédents de tabagisme pour la patiente, les sœurs, la mère, les grands-mères |
| Fréquence de voyage/nombre d'heures de vol par an/changements d'heure de plus de 3 heures (décalage horaire) |
| Nature des règles (durée des menstrues, durée du cycle) |
| Âge biologique (nombre d'années depuis les premières menstrues) |
| Utilisation d'un moyen contraceptif |
| Consommation de drogues (illégales ou légales) |
| Indice de masse corporelle (actuel, le plus bas jamais atteint, le plus haut jamais atteint) |
| Antécédents de polypes |
| Antécédents de déséquilibre hormonal |
| Antécédents d'aménorrhée |
| Antécédents de troubles de l'alimentation |
| Consommation d'alcool par la patiente, les sœurs, la mère, la grand-mère, les grands-mères |
| Détails de la grossesse de la mère avec la patiente : consommation de drogues, tabagisme, alcool, niveau de stress, exposition aux matières plastiques (ex. : Tupperware), composition de l'alimentation (voir ci-dessous) |
| Sommeil : nombre d'heures par nuit, en continu/en général |
| Alimentation : viande, produits biologiques, légumes, vitamines ou autres suppléments, produits laitiers (entiers ou allégés), café/thé, acide folique, sucre (complexe, artificiel, simple), aliments transformés par rapport à la cuisine maison. |
| Exposition aux matières plastiques : utilisation de plastique avec un four à micro-ondes, utilisation de plastique pour cuisiner, utilisation de plastique pour conserver les aliments, eau dans des bouteilles en plastique ou tasses à café en plastique. |
| Consommation d'eau : quantité par jour, format : directement du robinet, eau en bouteille (plastique ou bouteille), filtrée (type Britta/Pur) |
| Antécédents de résidence à partir de la grossesse de la mère : lieu/durée |
| Exposition environnementale à des toxines potentielles pour différentes régions (extrait des bases de données de surveillance gouvernementales) |
| Paramètres de santé : maladies auto-immunes, maladies chroniques/état de santé |
| Antécédents de chirurgie pelvienne |
| Nombre total de radiographies pelviennes |
| Antécédents d'infections sexuellement transmissibles : type/traitement/résultats |
| Taux d'hormones de la reproduction : hormone folliculostimulante, hormone anti-mullérienne, œstrogène, progestérone |
| Stress |
| Épaisseur et type d'endomètre tout au long du cycle menstruel. |
| Âge |
| Taille |
| Antécédents et détails du traitement de l'infertilité : antécédents de stimulation hormonale, marque de médicaments utilisés, nombre de follicules basaux antaux, nombre de follicules après stimulation avec différents protocoles, nombre/qualité/état des ovocytes prélevés/profil de développement des embryons résultant de l'insémination in vitro (naturelle ou ICSI), détails de la procédure FIV (quelle clinique, médecin/embryologiste en clinique, accouchement assisté, ovules frais ou dégelés, transfert embryonnaire (détection et direction du sang sur la sonde/lexium à ultrason) |
| Nausées matinales pendant la grossesse |
| Taille des seins avant, pendant et après la grossesse |
| Antécédents de kystes ovariens |
| Jumeaux ou frères et sœurs à partir de naissances multiples (monozygotiques ou dizygotiques) |
| Iinfertilité de facteur masculin pour le partenaire reproducteur : analyse du sperme (numération, motilité, morphologie), vasectomie, cancer masculin, tabagisme, alcool, alimentation, ITS |
; et
c) réaliser une étude d'association basée sur les résultats des étapes (a) et (b) en utilisant des variables de prédiction pondérées pour évaluer l'infertilité et/ou la qualité des œufs chez le mammifère post-pubère.

2. Procédé selon la revendication 1, comprenant en outre l'étape consistant à réaliser un test sur un produit génique anormal.

3. Procédé selon la revendication 1, la variation étant choisie dans le groupe constitué par : un polymorphisme nucléotidique unique ; une délétion ; une insertion ; un réarrangement ; une variation du nombre de copies ; et une combinaison de ceux-ci.

4. Procédé selon la revendication 2, le produit génique étant un produit d'un gène d'effet maternel.

5. Procédé selon la revendication 4, le gène d'effet maternel étant un gène du Tableau 1.

6. Procédé selon la revendication 2, le produit génique étant l'ARN ou une protéine.

7. Procédé selon la revendication 4, le test comprenant les étapes consistant à déterminer une quantité de produit génique et à comparer la quantité déterminée à une référence.
